# EUROPEAN PATENT SPECIFICATION

(11) **EP 4 597 102 B1**
(45) Date of publication and mention of the grant of the patent: **14.01.2026**
(21) Application number: 25154676.8
(22) Date of filing: 29.01.2025
(51) Int. Cl.: G01N 33/493

(54) **URINE SAMPLE ANALYSIS SYSTEM AND SAMPLE ANALYSIS METHOD THEREOF**
URINPROBENANALYSESYSTEM UND URINPROBENANALYSEVERFAHREN
SYSTÈME D'ANALYSE D'ÉCHANTILLONS D'URINE ET MÉTHODE D'ANALYSE D'ÉCHANTILLONS D'URINE

(30) Priority: 02.02.2024 JP 2024014789
(43) Date of publication of application: 06.08.2025
(73) Proprietor: SYSMEX CORPORATION, Kobe-shi Hyogo 651-0073 (JP)
(72) Inventor: Nakamichi, Daisuke, Kobe-shi, Hyogo, 651-0073 (JP); Tanaka, Masamichi, Kobe-shi, Hyogo, 651-0073 (JP); Koike, Hiroki, Kobe-shi, Hyogo, 651-0073 (JP); Senda, Go, Kobe-shi, Hyogo, 651-0073 (JP); Takehara, Hisato, Kobe-shi, Hyogo, 651-0073 (JP); Sakamoto, Mitsumasa, Kobe-shi, Hyogo, 651-0073 (JP); Matsui, Yousuke, Kobe-shi, Hyogo, 651-0073 (JP); Watanabe, Toshio, Kobe-shi, Hyogo, 651-0073 (JP)
(74) Representative: Hoffmann Eitle

(56) References cited:
- US-A1- 2010 312 137
- US-A1- 2018 017 480

## Description

### CROSS REFERENCE TO RELATED APPLICATIONS

This application claims priority from prior Japanese Patent Application No. 2024-014789, filed on February 2, 2024, entitled "SAMPLE ANALYSIS METHOD AND SAMPLE ANALYSIS SYSTEM".

### FIELD OF THE INVENTION

The present invention relates to a sample analysis system and a sample analysis method.

### BACKGROUND OF THE INVENTION

In urinalysis, urinary qualitative test to examine chemical compositions of glucose, protein, etc. in urine and urine sediment test to analyze particles of red blood cells, white blood cells, and bacteria, etc. in urine are known. In addition, the urine sediment test is known to be a method of capturing an image and a method of using a flow cytometer.

Conventionally, urinary qualitative test and urine sediment test have been combined to achieve accurate urine testing. The Japanese Laid-Open Patent Publication No.H6-138120 describes a urine particle analyzer in which a qualitative analysis section and a urine sediment analysis section are provided, samples are analyzed in the qualitative analysis section, and measurement conditions for analyzing urine sediment components in sample solutions are automatically changed for each sample according to whether analysis results of the qualitative analysis section are positive or negative. As an example, when protein is positive by qualitative analysis, it is described that the measurement time of the sediment analysis section is lengthened.

Urinalysis is a laboratory test used as medical checkups and screening tests for outpatients and hospitalized patients. Since the number of samples to be processed is large, efficient processes by an analyzer are required. However, in the processes by the analyzer described in Japanese Laid-Open Patent Publication No.H6-138120, since measurement conditions of the urine sediment section are changed between two alternative conditions according to whether an analysis result of the qualitative analysis section is positive or negative, it was difficult to efficiently measure urine sediments. US 2010/312137 A1 further describes a urine analysis device and method comprising a first and second analysers of different type, wherein a sample amount to be analysed by the second analyser depends on a measurement of the first analyser.

Regardless of the urinalysis system, including the urinary qualitative test and urine sediment test, the above-mentioned problems were found in other analysis systems capable of multi-step analysis with different methods and conduct further detailed analysis according to results of the previous analysis. It was desirable to solve them.

In view of this problem, it is an object of the present invention to provide a sample analysis system and a sample analysis method in which reliable analytical results can be efficiently provided using a plurality of analyzers of different measurement methods.

### SUMMARY OF THE INVENTION

The scope of the present invention is defined solely by the appended claims, and is not affected to any degree by the statements within this summary.

A sample analysis system of the present invention comprises: a plurality of analyzers including a first analyzer and a second analyzer, wherein the first analyzer and the second analyzer employ different measurement methods, the first analyzer being configured to measure a urine sample using a test strip that changes its color according to an amount of an analyte in the urine sample and obtain, based on the color of the test strip, a first measurement result representing the amount of the analyte in three or more levels, wherein the three or more levels consist of (i) a first level indicating that the amount of the analyte is a normal level, and (ii) a plurality of second levels indicating that the amount of the analyte is higher than the first level; and a management device configured to obtain the first measurement result, and determine a sample amount to be measured by the second analyzer based on the level represented by the first measurement result, wherein the sample amount corresponding to the lowest level of the plurality of second levels is configurable to be greater than the sample amount corresponding to the highest level of the plurality of second levels,
wherein the second analyzer is configured to measure the urine sample having the sample amount determined by the management device and obtain a second measurement result of the urine sample.

### [Effect of the invention]

According to the present invention, reliable analytical results can be efficiently provided using a plurality of analyzer in which the measurement methods differ.

### BRIEF DESCRIPTION OF THE DRAWING

FIG. 1 is a front view schematically showing the configuration of the sample analysis system according to the embodiment.
FIG. 2 is a block diagram illustrating a functional configuration of a urine qualitative analyzer, a urine particle analyzer, and a imaging analyzer according to an embodiment.
FIG. 3 is a block diagram illustrating a functional configuration of a management device according to an embodiment.
FIG. 4 is a diagram schematically showing the configuration of an optical measurement unit according to the embodiment.
FIG. 5 is a diagram schematically showing the configuration of the imaging unit according to the embodiment.
FIG. 6 is a diagram showing the measurement items of the urine qualitative analyzer, the measurement items of the urine particle analyzer, and the measurement items of the imaging analyzer according to the embodiment.
FIG. 7 is a diagram illustrating a rule for additional measurements where the first analyzer is urine qualitative analyzer and the second analyzer is urine particle analyzer, according to the embodiment.
Fig. 8 is a diagram illustrating a rule for additional measurement when the first analyzer according to the embodiment is urine qualitative analyzer and the second analyzer is imaging analyzer.
FIG. 9 is a diagram illustrating the rules for additional measurements when the first analyzer is urine particle analyzer and the second analyzer is imaging analyzer, according to the embodiment.
FIG. 10 is a diagram schematically showing a rule list screen showing a rule list of additional measurements according to the embodiment.
FIG. 11 is a diagram schematically showing a rule setting screen for setting a rule for additional measurement according to the embodiment.
FIG. 12 is a diagram schematically showing a termination condition setting screen for setting a termination condition according to the embodiment.
FIG. 13 is a diagram schematically showing a rule setting screen in which the rule for additional measurement regarding "Protein" as shown in FIG. 7 is set according to the embodiment.
FIG. 14 is a diagram schematically showing a termination condition setting screen in which the termination condition regarding "Protein" as shown in FIG. 7 is set according to the embodiment.
FIG. 15 is a diagram schematically showing a rule setting screen in which the rule of additional measurement regarding "Specific Gravity" as shown in FIG. 7 is set according to the embodiment.
FIG. 16 is a diagram schematically showing a rule setting screen in which the rule for additional measurement regarding "Bilirubin" as shown in FIG. 8 is set according to the embodiment.
FIG. 17 is a diagram schematically showing a termination condition setting screen in which the termination condition regarding "Bilirubin" as shown in FIG. 8 is set according to the embodiment.
FIG. 18 is a diagram schematically showing a rule setting screen in which the rule of the additional measurement regarding "Atypical Cell" as shown in FIG. 9 is set according to the embodiment.
FIG. 19 is a diagram schematically showing a termination condition setting screen in which the termination condition regarding "Atypical Cell" as shown in FIG. 9 is set according to the embodiment.
FIG. 20 is a diagram schematically showing a results list screen for displaying a list of measurement results according to the embodiment.
FIG. 21 is a diagram schematically showing a detailed result screen for displaying a detailed measurement result according to the embodiment.
FIG. 22 is a diagram schematically showing a detailed result screen for displaying a detailed measurement result according to the embodiment.
FIG. 23 is a diagram schematically showing a detailed result screen for displaying a detailed measurement result according to the embodiment.
FIG. 24 is a flowchart showing a process of setting a rule for additional measurement according to the embodiment.
FIG. 25 is a flowchart illustrating a process of a first analyzer and a management device according to an embodiment.
FIG. 26 is a diagram schematically illustrating the renewal and entry of an order of second analyzer in accordance with an embodiment.
FIG. 27 is a flowchart illustrating a process of a second analyzer and a management device according to an embodiment.
FIG. 28 is a flowchart illustrating a process of measuring urine qualitative analyzer, a urine particle analyzer, and a imaging analyzer according to an embodiment.
FIG. 29 is a flowchart showing the process of the second analyzer and the management device according to the modification example 1.
FIG. 30 is a flowchart showing the process of the second analyzer according to the modification example 2.
FIG. 31 is a flowchart showing the process of the second analyzer according to the modification example 3.

### DETAILED DESCRIPTION

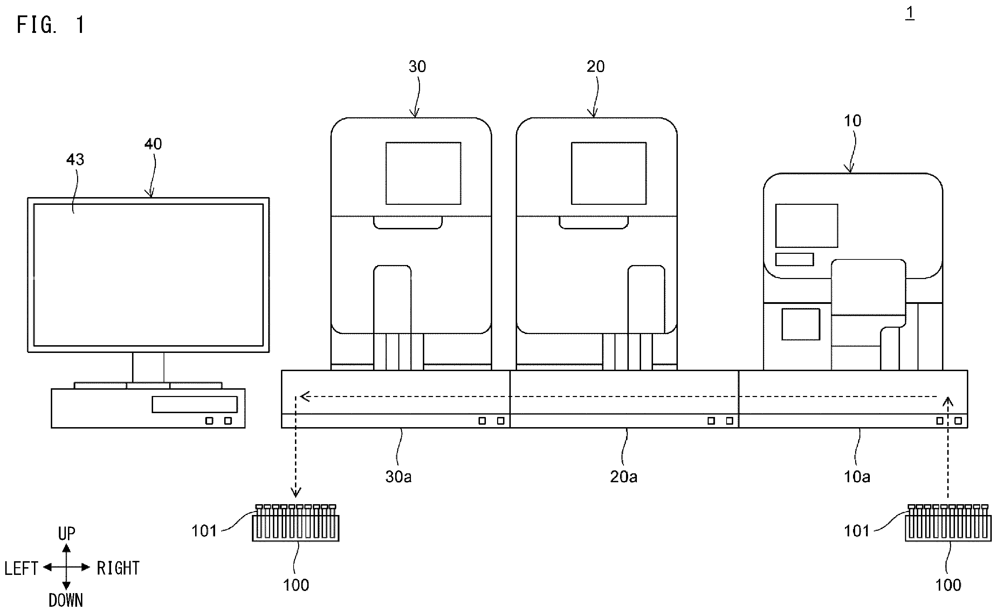

Fig. 1 is a front view schematically showing the configuration of the sample analysis system 1. Figure 1 shows the vertical and horizontal directions.

The sample analysis system 1 is a system for analyzing urine samples. In addition to excreted urine, the urine sample to be analyzed includes urine collected from the body, such as urine in the bladder. The sample analysis system 1 includes a urine qualitative analyzer 10, a urine particle analyzer 20, a imaging analyzer 30, and a management device 40.

The urine qualitative analyzer 10 is a device for analyzing chemical constituents in urine samples for several urinary qualitative measures. The urine particle analyzer 20 is a urinary particle analyzer for analyzing particle in urine samples for several measures of urine sediment using a flow cytometer. The imaging analyzer 30 is a urinary particle analyzer for analyzing particle in a urine sample for a plurality of measurement items using images obtained and captured images of the particle contained in the urine sample. In the analysis in the imaging analyzer 30, at least the measurement results corresponding to the measurement items may be obtained, and further analysis based on the measurement results (e.g., suggestion of possible diseases) may not be performed.

The urine qualitative analyzer 10, the urine particle analyzer 20 and the imaging analyzer 30, respectively, includes transport devices 10a, 20a, 30a on the front side. The transport devices 10a, 20a, 30a are connected to each other so that each transport device conveys sample racks 100 holding a plurality of sample containers 101 to the adjacent transport device. The sample container 101 houses the sample collected from a subject.

The operator holds the sample container 101 containing the sample to be inspected in the sample rack 100 and sets the sample rack 100 in the right end area of the transport device 10a. Transport devices 10a, 20a, 30a convey sample racks 100 sequentially to the urine qualitative analyzer 10, the urine particle analyzer 20 and the imaging analyzer 30. The urine qualitative analyzer 10 reads the sample **ID** from the sample container 101 and aspirates the sample in the sample container 101 and measures the urine quality of the aspirated sample to produce a measurement result. The urine particle analyzer 20 reads the sample **ID** from the sample container 101 and aspirates the sample in the sample container 101 and measures the urine particle for the aspirated sample to produce a measurement result. The imaging analyzer 30 reads the sample **ID** from the sample container 101 and aspirates the sample in the sample container 101 to capture the aspirated sample to produce a measurement.

When the urine qualitative analyzer 10, the urine particle analyzer 20, and the imaging analyzer 30 complete the required measurement process for all of the samples held in the sample rack 100, the sample rack 100 is conveyed to the left end area of the transport device 30a. The operator removes the sample rack 100 that has been processed from the transport device 30a.

The management device 40 receives and stores the measurement results obtained by the urine qualitative analyzer 10, the measurement results obtained by the urine particle analyzer 20, and the measurement results obtained by the imaging analyzer 30, and displays the measurement results in a display 43 according to an instruction of the operator.

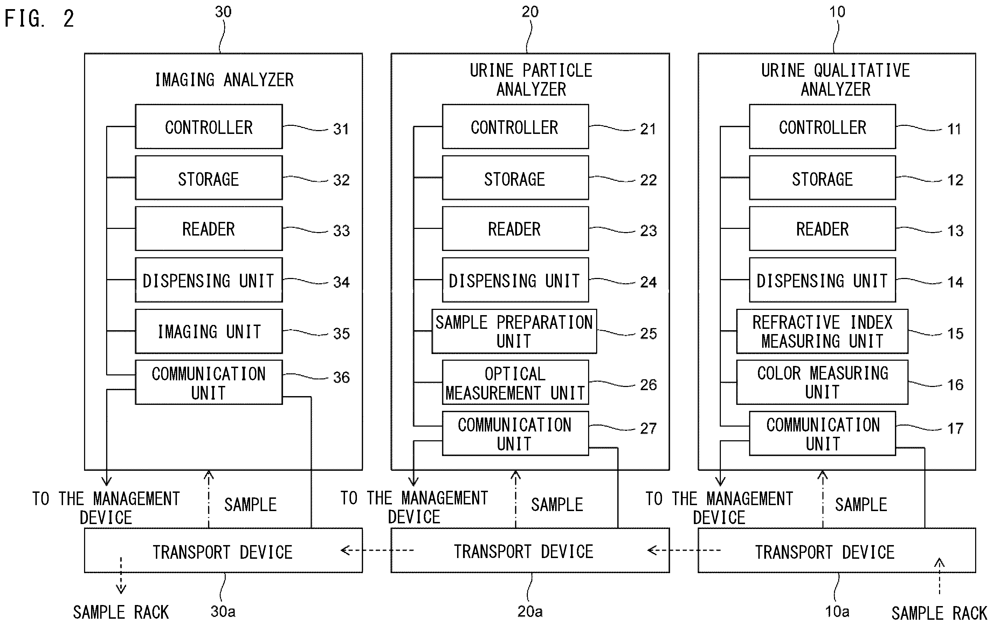

Fig. 2 is a block diagram showing the functional configuration of the urine qualitative analyzer 10, the urine particle analyzer 20, and the imaging analyzer 30.

The urine qualitative analyzer 10 includes a controller 11, a storage 12, a reader 13, a dispensing unit 14, a refractive index measuring unit 15, a color measuring unit 16, and a communication unit 17.

The controller 11, for example, is configured with a CPU or FPGA. The controller 11 performs measurement and analysis of the sample by executing a computer program stored in the storage 12. The storage 12 is configured with, for example, an SSD or HDD. In the storage 12, the measurement data acquired by the refractive index measuring unit 15 and the color measuring unit 16 and the measurement result (analysis result) generated by the analysis of the measurement data is stored.

The reader 13 reads the sample ID for individually identifying the sample from the sample container 101. If a bar code label is printed containing the sample ID as information on the sample container 101, the reader 13 is configured with, for example, a bar code reader. The dispensing unit 14 inserts an aspiration tube from above the sample container 101 conveyed by the transport device 10a, releases air from the tip of the aspiration tube to agitate the sample in the sample container 101. The dispensing unit 14 aspirates the sample in the sample container 101 and supplies the aspirated sample to the refractive index measuring unit 15 and the color measuring unit 16.

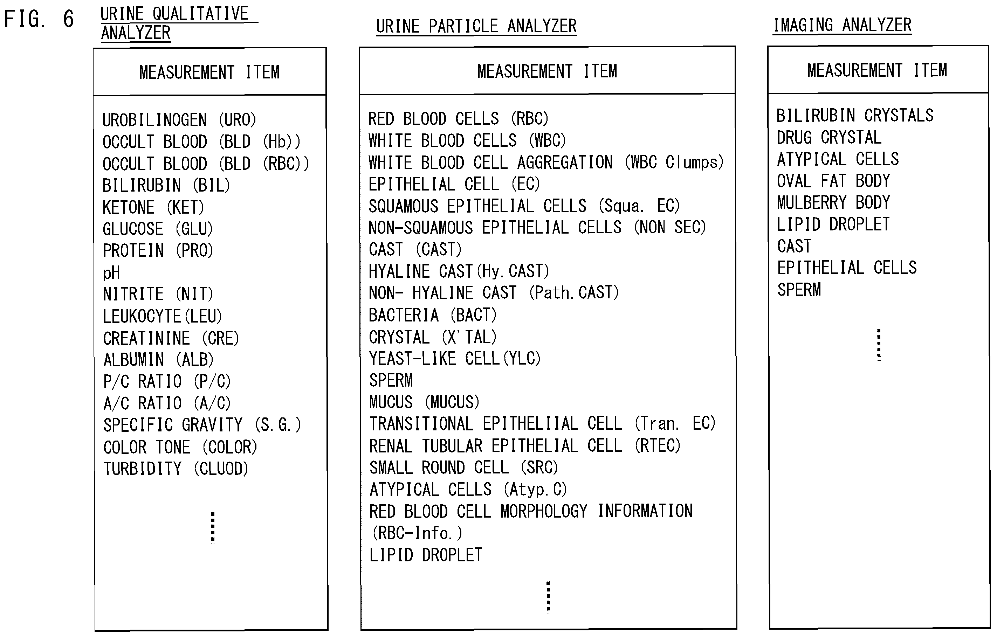

The refractive index measuring unit 15 includes a measuring container to accommodate the sample supplied by the dispensing unit 14. The refractive index of the sample in the measuring container is measured using a prism. The refractive index measuring unit 15 irradiates the sample with light and measures the transmitted light and the scattered light. The controller 11 obtains the measurement results of the specific gravity, color tone and turbidity based on the measurement data obtained by the refractive index measuring unit 15. The color measuring unit 16 removes the test strip from the test strip feeder and attaches the sample supplied by the dispensing unit 14 to the test strip. The color measuring unit 16 irradiates the test strip to which the sample is attached with light and measures the color of the test strip with a color sensor. The controller 11 acquires various measurement results based on the measurement data acquired by the color measuring unit 16. The measured items of the urine qualitative analyzer 10 will be described later with reference to FIG. 6.

The communication unit 17 includes a connection terminal based on the Ethernet standard, and a connection terminal based on a serial communication standard such as USB. The communication unit 17 communicates between the management device 40 via a cable based on the Ethernet standard, and performs communication between the transport device 10a via a cable based on the serial communication standard such as USB. The controller 11 controls the transport device 10a via the communication unit 17.

As the specific configuration of the urine qualitative analyzer 10, the urine qualitative analyzer disclosed in U.S. Patent Application Publication No. 2016/0061851 may be adopted for example.

The urine particle analyzer 20 includes a controller 21, a storage 22, a reader 23, a dispensing unit 24, a sample preparation unit 25, an optical measuring unit 26, and a communication unit 27.

The controller 21, for example, is configured with a CPU or FPGA. The controller 21 performs measurement and analysis of the sample by executing the computer program stored in the storage 22. The storage 22, for example, is configured with an SSD or HDD. In the storage 22, the measurement data acquired by the optical measurement unit 26 and the measurement result generated by the analysis of the measurement data (analysis result) is stored.

The reader 23 reads the sample **ID** from the sample container 101. The reader 23 is configured by, for example, a bar code reader. The dispensing unit 24 inserts an aspiration tube from above the sample container 101 conveyed by the transport device 20a. The dispensing unit 24 agitates the sample in the sample container 101 by discharging air from the tip of the aspiration tube. Then, the dispensing unit 24 aspirates the sample in the sample container 101 and supplies it to the sample preparation unit 25.

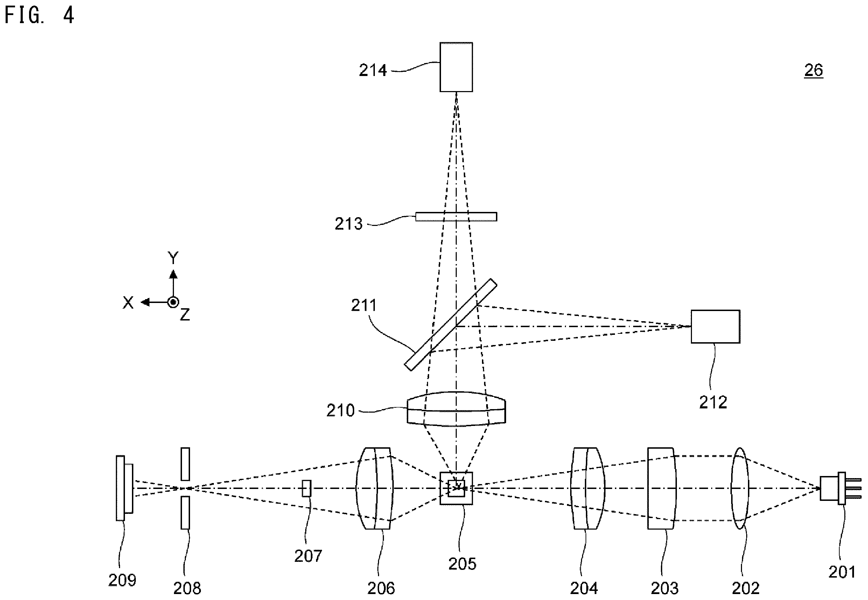

The sample preparation unit 25 includes a reaction chamber in which the sample is supplied by the dispensing unit 24. The sample preparation unit 25 prepares the measurement sample by mixing the sample and the reagent in the reaction chamber. Optical measuring unit 26 is a flow cytometer for measuring a measurement sample based on the flow cytometry method. The optical measurement unit 26 will be described with reference to FIG. 4 later. Controller 21 acquires various measurement results based on the measurement data acquired by the optical measurement unit 26. The measured items of the urine particle analyzer 20 will be described later with reference to FIG 6.

The communication unit 27 includes a connection terminal based on the Ethernet standard. The communication unit 27 includes a connection terminal based on a serial communication standard such as USB. The communication unit 27 communicates between the management device 40 via a cable based on the Ethernet standard, and performs communication between the transport device 20a via a cable based on the serial communication standard such as USB. The controller 21 controls the transport device 20a via the communication unit 27.

As the specific configuration of the urine particle analyzer 20, the urinary particle analyzer disclosed in U.S. Patent Application Publication No. 2016/0061851 may be adopted for example.

The imaging analyzer 30 includes a controller 31, a storage 32, a reader 33, a dispensing unit 34, an imaging unit 35, and a communication unit 36.

Controller 31 is configured with, for example, a CPU or FPGA. Controller 31 performs measurement (capturing of image) and analysis of the sample by executing the computer program stored in the storage 32. Storage 32 is configured with, for example, an SSD or HDD. In the storage 32, the measurement data (image) acquired by the imaging unit 35 and the analysis result generated by the analysis of the measurement data (image) is stored.

The reader 33 reads the sample ID from the sample container 101. The reader 33 is configured with, for example, a bar code reader. Dispensing unit 34 inserts an aspiration tube from above the sample container 101 conveyed by the transport device 30a, and agitates the sample in the sample container 101 by discharging air from the tip of the aspiration tube. Thereafter, the dispensing unit 34 aspirates the sample in the sample container 101, and supplies it to the imaging unit 35.

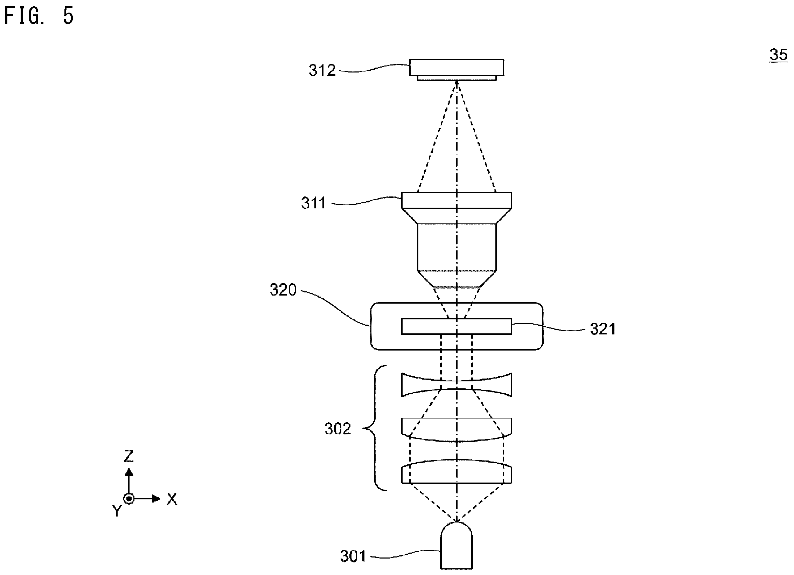

Imaging unit 35 includes a cell in which the sample is supplied by the dispensing unit 34. Imaging unit 35 images the particle in the sample accommodated in the cell. The imaging unit 35 will be described with reference to FIG. 5 later. Controller 31 acquires various measurement results based on the measurement data acquired by the imaging unit 35. The measured items of the imaging analyzer 30 will be described with reference to FIG. 6 later.

Communication unit 36 includes a connection terminal based on the Ethernet standard, and a connection terminal based on a serial communication standard such as USB. Communication unit 36 communicates between the management device 40 via a cable based on the Ethernet standard and performs communication between the transport device 30a via a cable based on the serial communication standard such as USB. Controller 31 controls the transport device 30a via the communication unit 36.

As the specific configuration of the imaging analyzer 30, the imaging device disclosed in U.S. Patent Application Publication No. 2018/0017480 may be applied for example. The content of this document is incorporated by reference as part of the specification.

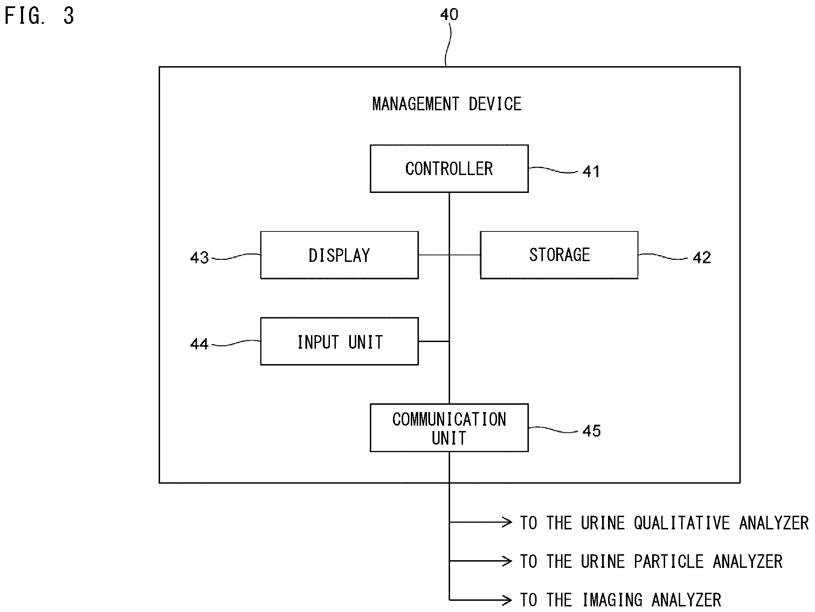

FIG. 3 is a block diagram showing the functional configuration of the management device 40.

The management device 40 includes a controller 41, a storage 42, the display 43, an input unit 44, and a communication unit 45.

The controller 41 is configured with, for example, a CPU. The controller 41 executes a computer program stored in the storage 42 to perform various processes such as management of measurement results. Storage 42 is configured with, for example, an SSD or HDD. The storage 42 stores the measured results transmitted from the urine qualitative analyzer 10, urine particle analyzer 20 and the imaging analyzer 30.

The display 43 is configured with, for example, a liquid crystal display or an organic **EL** display. An input unit 44 is configured with a mouse or a keyboard for example. The display 43 and the input unit 44 may be integrally configured, for example, as a touch panel type display.

The communication unit 45 includes a connection terminal based on the Ethernet standard. The communication unit 45 communicates with the urine qualitative analyzer 10, the urine particle analyzer 20 and the imaging analyzer 30 via a cable based on the Ethernet standard. The controller 41 transmits various instruction information, via the communication unit 45, to the urine qualitative analyzer 10, urine particle analyzer 20 and the imaging analyzer 30, and receives the measured results from these devices.

As the specific configuration of the management device 40, the information processing apparatus disclosed in U.S. Patent Application Publication No. 2017/0153221 may be applied for example.

FIG. 4 is a diagram schematically showing the configuration of the optical measurement unit 26. In FIG. 4, the X, Y, Z axes, which are perpendicular to each other, are illustrated for easy reference.

The laser light source 201 emits laser light of a predetermined wavelength in the X-axis direction. The collimator lens 202 converts the laser light emitted from the laser light source 201 into parallel light. The cylindrical lens 203 converges the laser light transmitted through the collimator lens 202 only in the Y-axis direction. The condenser lens 204 focuses the laser light transmitted through the cylindrical lens 203 in the Y-axis and Z-axis directions. As a result, the laser light emitted from the laser light source 201 is irradiated in an elongated beam shape in the Y-axis direction onto the measurement sample flowing in the Z-axis direction within the flow cell 205. When the laser light is irradiated onto the formed elements in the measurement sample, forward scattered light is generated in front of the flow cell 205, and side scattered light and fluorescence are generated on the side of the flow cell 205.

The condenser lens 206 focuses the forward scattered light onto the position of the pinhole 208. Of the light emitted from the laser light source 201, the laser light that has passed through the flow cell 205 without being irradiated onto the formed elements in the measurement sample is focused by the condenser lens 206 and then blocked by the beam stopper 207 so as not to enter the light receiving unit 209. The light receiving unit 209 receives the forward scattered light that has passed through the pinhole 208 and outputs a signal based on the intensity of the received forward scattered light. The light receiving unit 209 is, for example, a photodiode.

The condenser lens 210 focuses the side scattered light and fluorescence. The dichroic mirror 211 reflects the side scattered light transmitted through the condenser lens 210 and transmits the fluorescence transmitted through the condenser lens 210.

The light receiving unit 212 receives the side scattered light reflected by the dichroic mirror 211 and outputs a signal based on the intensity of the received side scattered light. The light receiving unit 212 is, for example, a photomultiplier. The spectroscopic filter 213 transmits fluorescence of a predetermined wavelength among the fluorescence transmitted through the dichroic mirror 211. The light receiving unit 214 receives the fluorescence transmitted through the spectroscopic filter 213 and outputs a signal based on the intensity of the received fluorescence. The light receiving unit 214 is, for example, a photomultiplier.

The signals output by the light receiving units 209, 212, and 214 are subjected to predetermined processing and then stored as measurement data in the storage unit 22.

FIG. 5 is a diagram schematically showing the configuration of the imaging unit 35. In FIG. 5, the X, Y, Z axes, which are perpendicular to each other, are illustrated for easy reference. The Z-axis direction is vertical.

The light source 301 emits light of a predetermined wavelength. The light source 301 is, for example, configured with a light-emitting diode. The illumination optical system 302 is composed of multiple lenses and converts the light from the light source 301 into parallel light, which is then irradiated onto the imaging area of the objective lens 311. The objective lens 311 forms an image of the light irradiated onto the imaging area on the light-receiving surface of the image sensor 312. The objective lens 311 is driven in the optical axis direction (Z-axis direction) for focus adjustment. The image sensor 312 is, for example, a CCD image sensor or a CMOS image sensor. The stage 320 includes a cell 321. The cell 321 is a rectangular container made of a transparent material.

During imaging, each sample supplied from the dispensing unit 34 is introduced into the cell 321. The stage 320 is driven to move the cell 321 in the X-axis direction. During this time, the objective lens 311 is moved in the optical axis direction for focus adjustment. While the cell 321 is positioned in the imaging area of the objective lens 311 and moved in the X-axis direction, the image sensor 312 captures multiple images of the sample filled in the cell 321. As a result, a predetermined number of images (for example, 40 images) are obtained for the sample in the cell 321.

Fig. 6 is a diagram showing the measurement items of the urine qualitative analyzer 10, the measurement items of the urine particle analyzer 20, and the measurement items of the imaging analyzer 30.

As shown in FIG. 6, the urine qualitative analyzer 10, the urine particle analyzer 20, and the imaging analyzer 30 are all configured to acquire measurements for a plurality of measurement items.

When the sample **ID** is acquired by the reader 13, the controller 11 of the urine qualitative analyzer 10 transmits the order inquiry information including the sample **ID** to the management device 40. When the controller 41 of the management device 40 receives the order inquiry information from the urine qualitative analyzer 10, it transmits the order of the urine qualitative analyzer 10 (order of the urine qualitative) set for the sample **ID** to the urine qualitative analyzer 10. The order of urine qualitative test includes the sample **ID** of the sample to be tested, and the information that indicates which of the urinary qualitative test items to be measured, as shown in the table on the left in Fig. 6. When the controller 11 of the urine qualitative analyzer 10 receives the order of the urine qualitative test from the management device 40, the controller 11 performs a control to run the urine qualitative test of the sample of interest based on the order received, and generates the measurement results specified in the order of the urine qualitative test.

When the sample **ID** is acquired by the reader 23, the controller 21 of the urine particle analyzer 20 transmits the order inquiry information including the sample **ID to** the management device 40. When the controller 41 of the management device 40 receives the order inquiry information from the urine particle analyzer 20, it transmits the order of the urine particle analyzer 20 (the order of the urine sediment test) set corresponding to the sample **ID** to the urine particle analyzer 20. The order of the urine sediment test includes the sample **ID** of the sample to be tested, and the information that indicates which of the urine sediment test items to be measured, as shown in the middle chart in Fig. 6. When the controller 21 of the urine particle analyzer 20 receives the order of the urine sediment test from the management device 40, the controller 21 performs a control to run the urine sediment test of the sample of interest based on the order received, and generates the measurement results specified in the order of the urine sediment test.

When the sample **ID** is acquired by the reader 33, the controller 31 of the imaging analyzer 30 transmits the order inquiry information including the sample **ID** to the management device 40. When the controller 41 of the management device 40 receives the order inquiry information from the imaging analyzer 30, it transmits the order of the imaging analyzer 30 (the order of image capturing) set corresponding to the sample **ID** to the imaging analyzer 30. The order of the image capture includes information such as the sample **ID** of the subject sample, and the information that indicates which of the measurement results to be measured by imaging, as shown in the table on the right side of FIG. 6. When the controller 31 of the imaging analyzer 30 receives an image capturing order from the management device 40, the controller 31 performs image capturing of the target sample based on the received order and generates the measurement results specified in the image capture order.

The orders of urine qualitative test, the order of urine sediment test and the order of image capturing are set in advance by an operator or the like in accordance with the decision of a physician and the like based on the patient information and the policy of the testing facility. The orders are stored in the storage 42 of the management device 40 or in the host computer with which the management device 40 can communicate.

In some embodiments, in addition to such a preset order, an additional order may be automatically set according to the measurement results of the sample.

For example, when samples are sequentially transported to the three analyzers (urine qualitative analyzer 10, urine particle analyzer 20, and imaging analyzer 30), it may be preferable to perform further measurements in the downstream analyzer (hereinafter referred to as the "second analyzer") based on the predetermined measurement results obtained by the upstream analyzer (hereinafter referred to as the "first analyzer"). In this case, even if the order for the second analyzer is not pre-set, an additional order for the second analyzer is automatically set for the measurement items related to the predetermined measurement results of the first analyzer, and the measurement by the second analyzer based on this order (hereinafter referred to as "additional measurement") is performed.

When the order for the second analyzer is pre-set, the additional order may be included in the pre-set order for the second analyzer, or the additional order may be set separately. For example, if the original order for the second analyzer specifies the measurement of item A and the additional measurement item is B, the original order for the second analyzer may be changed to an additional order specifying measurement items A and B, or an additional order specifying measurement item B may be set separately from the original order for the second analyzer.

The urine particle analyzer 20 can obtain the measurement results of all the measurement items of the urine particle analyzer 20 through a common measurement operation. As such, all the measurement items may be set as the additional order for the urine particle analyzer 20. Similarly, the imaging analyzer 30 can obtain the measurement results of all the measurement items of the imaging analyzer 30 through a common measurement operation. Therefore, all the measurement items may be set as the additional order for the imaging analyzer 30.

As a specific example, if protein is detected in the urine qualitative analyzer 10, an additional order for urine sediment test is automatically set for that sample. The measurement item "casts (CAST)" of the urine particle analyzer 20 related to the measurement item "protein (PRO)" of the urine qualitative analyzer 10 is designated as the additional measurement item, and an additional order including the designated measurement item is set. The urine particle analyzer 20 automatically performs the additional measurement, and the presence or absence of casts related to the detection of protein is examined.

When the protein detected in the urine qualitative analyzer 10 is a trace amount, it is assumed that the amount of the cast contained in the sample is small. In the additional measurement in the urine particle analyzer 20, it is preferable that the presence or absence of the cast is reliably tested by performing the measurement of a predetermined number of times. Therefore, in the embodiment, in order to increase the possibility of obtaining reliable measurement results, an order is set so that additional measurements are performed multiple times on the samples of the same sample ID. The measurement performed multiple times in the same measurement method in the additional measurement is hereinafter referred to as "multiple measurements".

Further, in the embodiment, when the multiple measurements are performed in the second analyzer, if a predetermined measurement result is obtained in the second analyzer, the series of the multiple measurements is terminated even in the middle of the multiple measurements. The condition for terminating multiple measurements is hereinafter referred to as "termination condition". Termination condition on additional order saves reagents, samples, times, etc. compared to the case of performing all number of measurements in multiple measurements.

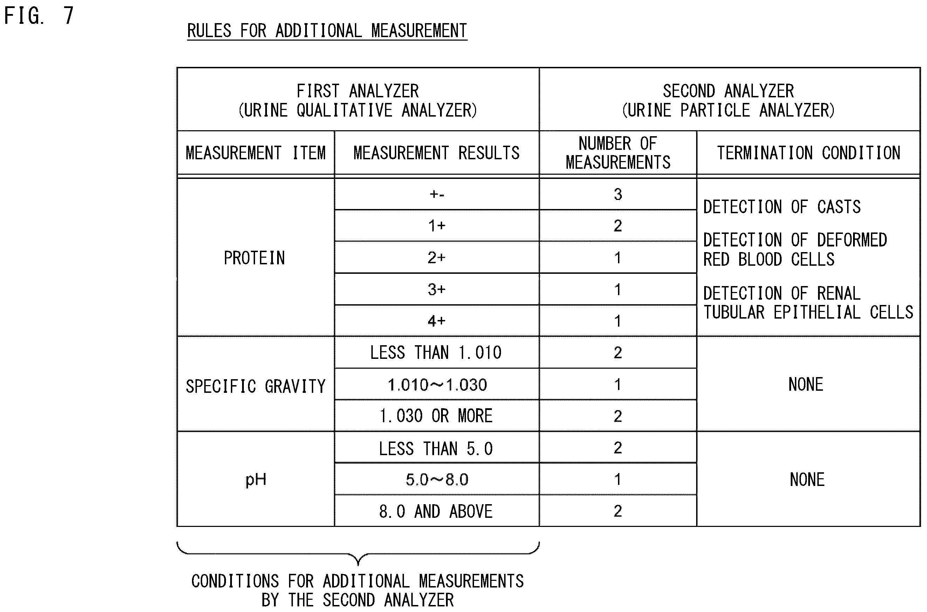

FIGS. 7 to 9 are diagrams illustrating the rules for additional measurements. FIGS. 7 to 9 show three, two, and five rules for additional measurements, respectively. In FIGS. 7 to 9, each rule for additional measurements includes the conditions for performing additional measurements by the second analyzer, the number of measurements for the additional measurements by the second analyzer, and the termination conditions for the multiple measurements by the second analyzer. Each condition for performing additional measurements by the second analyzer includes the measurement items of the first analyzer and the criteria corresponding to the measurement results of the measurement items.

FIG. 7 is a diagram illustrating the rules for additional measurements when the first analyzer is the urine qualitative analyzer 10 and the second analyzer is the urine particle analyzer 20.

When the measurement result of the measurement item "protein (PRO)" of urine qualitative analyzer 10 is "+-", "1+", "2+", "3+", or "4+", 3, 2, 1, 1, or 1 times of additional measurement is performed in urine particle analyzer 20, respectively. An additional order is set to include the measurement item "cast", "red cell morphology information (RBC-Info.)", and "renal tubular epithelial cells (RTEC)". In this case, the termination condition is set so that the series of multiple measurements is terminated when following condition is met in one of the measurement results obtained by each of the additional measurement:
- the measurement result of "cast" is more than a predetermined value
- the measurement result of "RBC-Info" indicates that the deformed red blood cells (Dysmorphic? or Mixed?) are present; or
- the measurement result of "RTEC" is more than a predetermined value. The termination condition may be set so that the series of multiple measurements is terminated when the two or more of the above listed conditions are met.

When the measurement result of the measurement item "specific gravity (S.G.)" in urine qualitative analyzer 10 is 1.010 or less, greater than 1.010 and less than 1.030, or 1.030 or more, then additional measurements are performed 2, 1, or 2 times in urine particle analyzer 20, respectively. An additional order is set to include all the measurement items. In this case, the measurement result of the measurement item "specific gravity (S.G.)" of the urine qualitative analyzer 10 is related to all measurement items of the urine particle analyzer 20. The termination condition is not set. The measurement of the predetermined number of times is performed.

If the measurement result of the measurement item "pH" in urine qualitative analyzer 10 is 5.0 or less, greater than 5.0 or less than 8.0, and 8.0 or more, then additional measurements are performed 2, 1, or 2 times in urine particle analyzer 20, respectively. An additional order is set to include all the measurement items. In this case, the measurement result of the measurement item "pH" of the urine qualitative analyzer 10 is related to all measurement items of the urine particle analyzer 20. The termination condition is not set. The measurement of the predetermined number of times is performed.

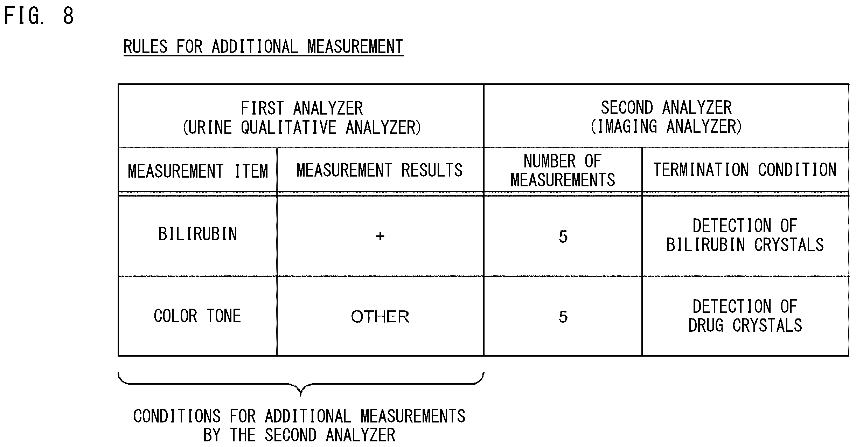

FIG. 8 is a diagram illustrating a rule for additional measurements where the first analyzer is the urine qualitative analyzer 10 and the second analyzer is the imaging analyzer 30.

If the measurement result of the urine qualitative analyzer 10 measurement item "bilirubin (BIL)" is "+", five additional measurements (image capturing) are performed at the imaging analyzer 30 and an additional order is set to include the measurement item "bilirubin crystals". In this case, the termination condition is set to terminate the multiple measurements if the measurement result of the measurement item "bilirubin crystal" is more than a predetermined value in any one of additional measurements of the imaging analyzer 30.

The measurement results of the measurement item "COLOR" in the urine qualitative analyzer 10 include "OTHER," "L YELLOW," "STRAW," "YELLOW," "AMBER," "RED," and "DK BROWN." If the measurement result of the measurement item "COLOR" in urine qualitative analyzer 10 is "OTHER", five additional measurements (image capturing) are performed in the imaging analyzer 30 and an additional order is set to include the measurement item "drug crystal". In this case, the termination condition is set to terminate the multiple measurements if the measurement result of the measurement item "drug crystal" is more than a predetermined value in any one of additional measurements of the imaging analyzer 30.

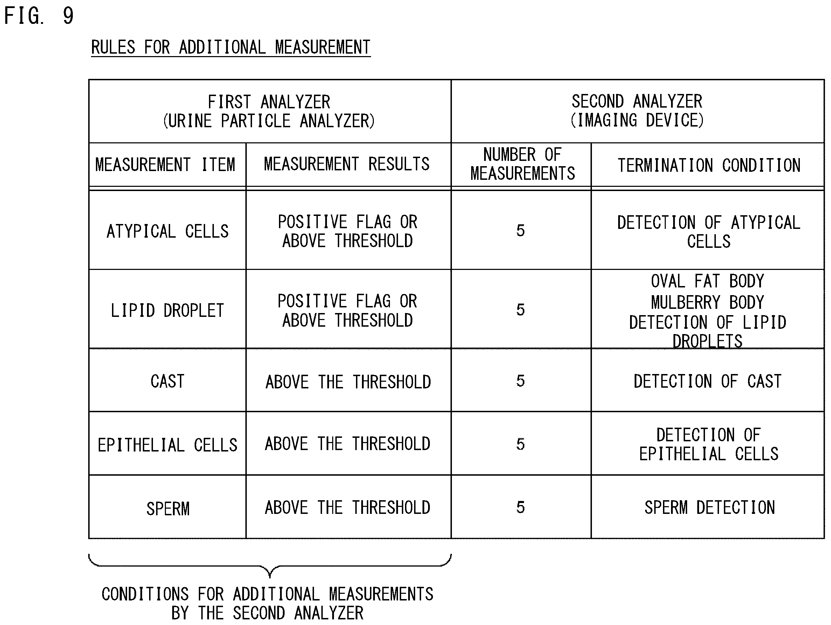

FIG. 9 is a diagram illustrating a rule for additional measurements where the first analyzer is urine particle analyzer 20 and the second analyzer is the imaging analyzer 30.

The measurement results of measurement item "atypical cell (Atyp.c)" of the urine particle analyzer 20 include a measurement value indicating the number per unit volume and a positive flag determined based on the measurement value. If the measurement result of the measurement item atypical cells (Atyp.c) of the urine particle analyzer 20 is positive flagged or greater than or equal to the threshold, five additional measurements (image capturing) are performed at the imaging analyzer 30 and an additional order is set to include the measurement item atypical cells. In this case, the termination condition is set to terminate the multiple measurements if the measurement result of the measurement item "atypical cell" is more than a predetermined value in any one of additional measurements of the imaging analyzer 30.

The measurement results of the measurement item "lipid droplet" in urine particle analyzer 20 include a measurement value indicating the number per unit volume and a positive flag determined based on the measurement value. If the measurement result of the measurement item "lipid droplet" of the urine particle analyzer 20 is positive flagged or greater than or equal to the threshold, five additional measurements (image capturing) are performed at the imaging analyzer 30 and an additional order is set to include the measurement items "oval fat bodies," "mulberry body," and "lipid droplet." In this case, the termination condition is set to terminate the multiple measurements if, in a measurement result of any one of additional measurements of the imaging analyzer 30, the measurement result of the measurement item "oval fat body" is equal to or greater than a predetermined value, the measurement result of the measurement item "mulberry body" is equal to or greater than a predetermined value, or the measurement result of the measurement item "lipid droplet" is equal to or greater than a predetermined value. The termination condition may be set to terminate the multiple measurements when the measurement results of two or three types of particles of these three types of particles are at least a predetermined value.

**If** the measurement result of the measurement item "cast" in urine particle analyzer 20 is greater than or equal to the threshold value, five additional measurements (image capturing) are performed in the imaging analyzer 30 and an additional order is set to include the measurement item "cast". **In** this case, the termination condition is set to terminate the multiple measurements if the measurement result of the measurement item "cast" is more than a predetermined value in any one of additional measurements of the imaging analyzer 30.

**If** the measurement result of measurement item "epithelial cells (EC)" of the urine particle analyzer 20 is greater than or equal to the threshold value, five additional measurements (image capturing) are performed at the imaging analyzer 30 and an additional order is set to include the measurement item "epithelial cell". **In** this case, the termination condition is set to terminate the multiple measurements if the measurement result of the measurement item "epithelial cell" is more than a predetermined value in any one of additional measurements of the imaging analyzer 30.

**If** the measurement result of the measurement item "sperm" of the urine particle analyzer 20 is greater than or equal to the threshold value, five additional measurements (image capturing) are performed at imaging analyzer 30 and an additional order is set to include the measurement item "sperm". **In** this case, the termination condition is set to terminate the multiple measurements if the measurement result of the measurement item "sperm" is more than a predetermined value in any one of the additional measurements of the imaging analyzer 30.

As shown in FIGS. 7 to 9, the first analyzer measurement items concerning the conditions for additional measurement by second analyzer and the measurement items concerning the termination condition of multiple measurements of second analyzer are related to each other. That is to say, according to the measurement result concerning the termination condition of the multiple measurements of the second analyzer, it is possible to estimate the same disease as the disease estimated from the measurement result of the first analyzer used for the determination of the necessity of the additional measurement by the second analyzer. For example, if the urine qualitative analyzer 10 (first analyzer) determines that the measurement result "protein (PRO)" is "+-", it can be inferred that a subject is suffering from a kidney-related disease. In this case, if the measurement result of the "casts (CAST)" item in the urine particle analyzer 20 (second analyzer) is equal to or greater than a predetermined value, it can be determined that the subject is highly likely to have a kidney-related disease.

In the embodiment, the measurement items of the first analyzer and the second analyzer are associated in the rules for additional measurements. The termination conditions for the multiple measurements by the second analyzer include obtaining the measurement results of the second analyzer that are suggested by the measurement results of the first analyzer used to determine whether to perform the tests by the second analyzer. This increases the likelihood of obtaining the measurement results of the second analyzer suggested by the measurement results of the first analyzer through multiple measurements. If the measurement results of the second analyzer are obtained, the measurements by the second analyzer are terminated, saving reagents consumed by the second analyzer, specimens supplied to the second analyzer, and the time required for the measurements.

A screen for setting the rules for additional measurements will be described.

A rule list screen 400, a rule setting screen 500, and a termination condition setting screen 600 shown below are displayed on the display 43 of the management device 40 by the controller 41 of the management device 40. When the operator of the management device 40 inputs an operation to the screen through the input unit 44, the controller 41 of the management device 40 executes processing in response to the input.

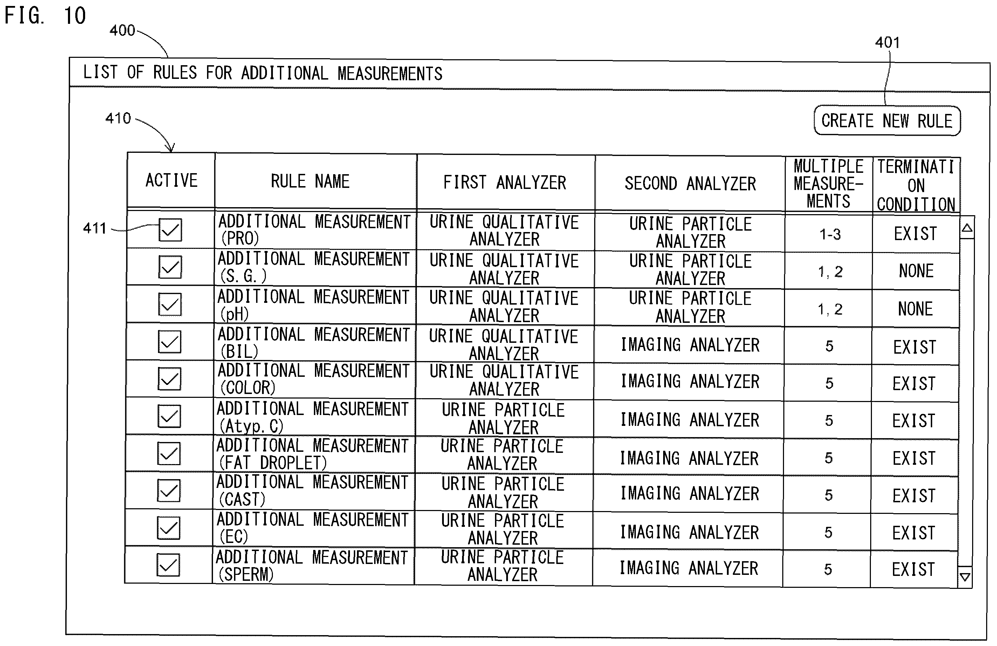

FIG. 10 is a diagram schematically showing the rule list screen 400 showing a rule list of additional measurements.

The list area 410 shows the rules for additional measurements in rows and includes the columns "Active", "Rule Name", "First Analyzer", "Second Analyzer", "Multiple Measurements" and "Termination Conditions."

The "Active" column includes a checkbox 411 that indicates whether the rule is active. The operator can set the rule to be active (checkbox checked) or inactive (checkbox unchecked) by operating the checkbox 411. The "Rule Name" column shows the name given to the rule by the operator. The "First Analyzer" column shows the name of the upstream analyzer that determines the conditions for performing additional measurements, and the "Second Analyzer" column shows the name of the downstream analyzer that performs the additional measurements. The "Multiple Measurements" column shows the number of measurements set for the second analyzer in the rule. The "Termination Conditions" column indicates whether conditions for terminating the multiple measurements are set in the rule.

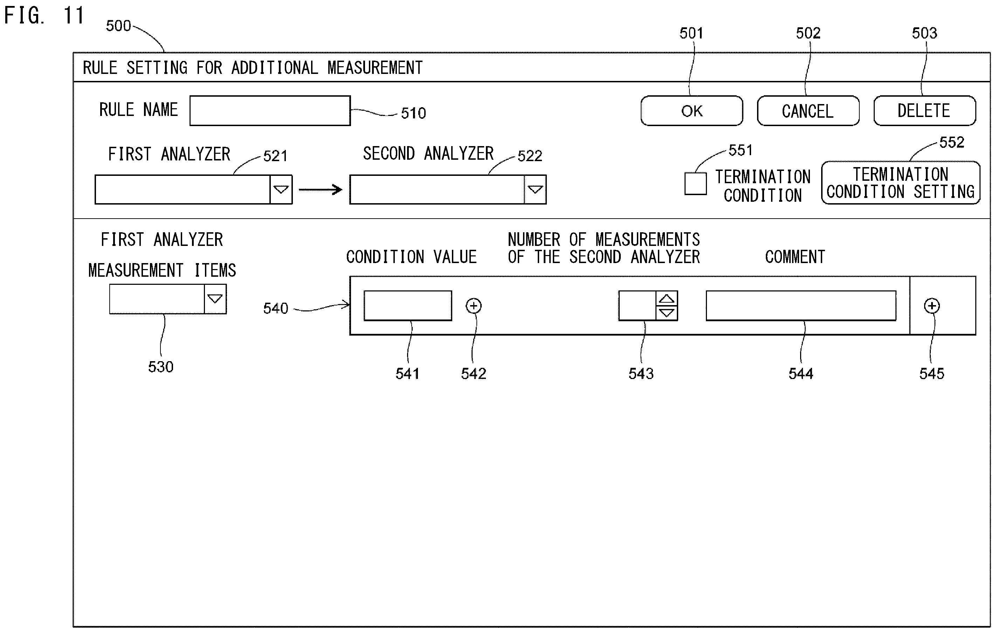

The operator operates New button 401 when creating a new rule. A rule setting screen 500 (see FIG. 11) is displayed on the display 43. The operator also operates the row in the list area 410 when editing an existing rule. Accordingly, the rule setting screen 500 including the rule of the operated row is displayed on the display 43.

FIG. 11 is a diagram schematically illustrating the rule setting screen 500 for setting rules for additional measurements.

The operator enters a name in the text box 510 that allows identification of the rule. The operator operates a pull-down menu 521 to enter one of the urine qualitative analyzer 10, the urine particle analyzer 20 and the imaging analyzer 30 as a first analyzer and to operate a pull-down menu 522 to enter one of the urine particle analyzer 20 and the imaging analyzer 30 as a second analyzer.

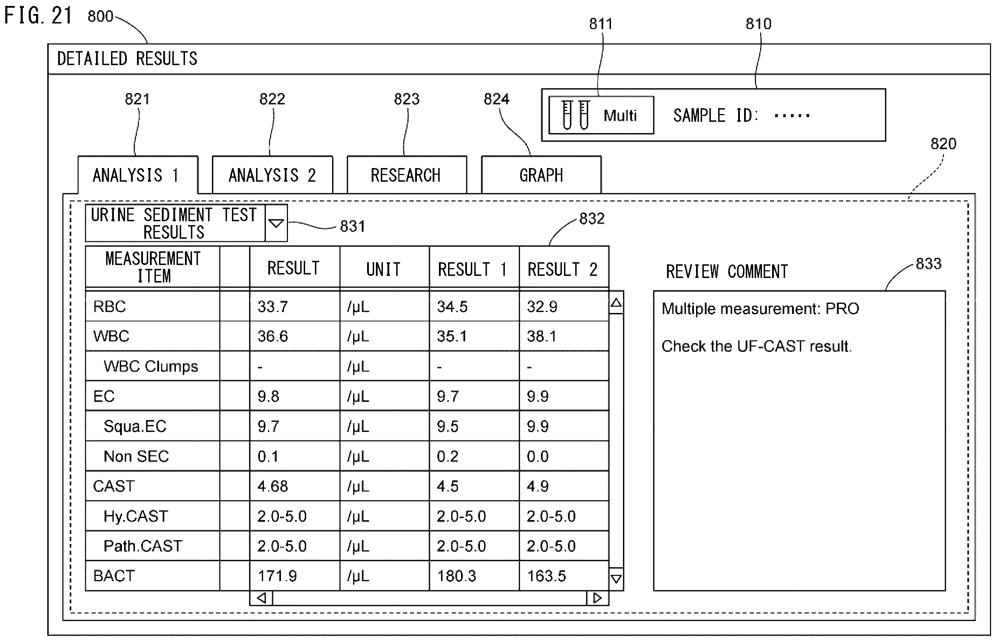

The operator operates the pull-down menu 530 to input the measurement item of the first analyzer to determine the necessity of additional measurements and inputs the condition value of the measurement item of the first analyzer in the text box 541. When multiple condition values are used, the operator can operate the add button 542 to add and display the text box 541. The operator inputs the number of measurements to be performed by the second analyzer in the text box 543. The operator inputs "1" in the text box 543 when configuring the number of measurement to be one time. Operator inputs a value of 2 or more in the text box 543 when configuring the number of measurements to be multiple times. The operator inputs a comment to be displayed on the detailed result screen 800 (see FIG. 21) when the measurement result of the first analyzer meets the condition value in the text box 544. If the operator wants to add more rules for the measurement items of the first analyzer, they operate the add button 545. Another condition area 540 is thereby added.

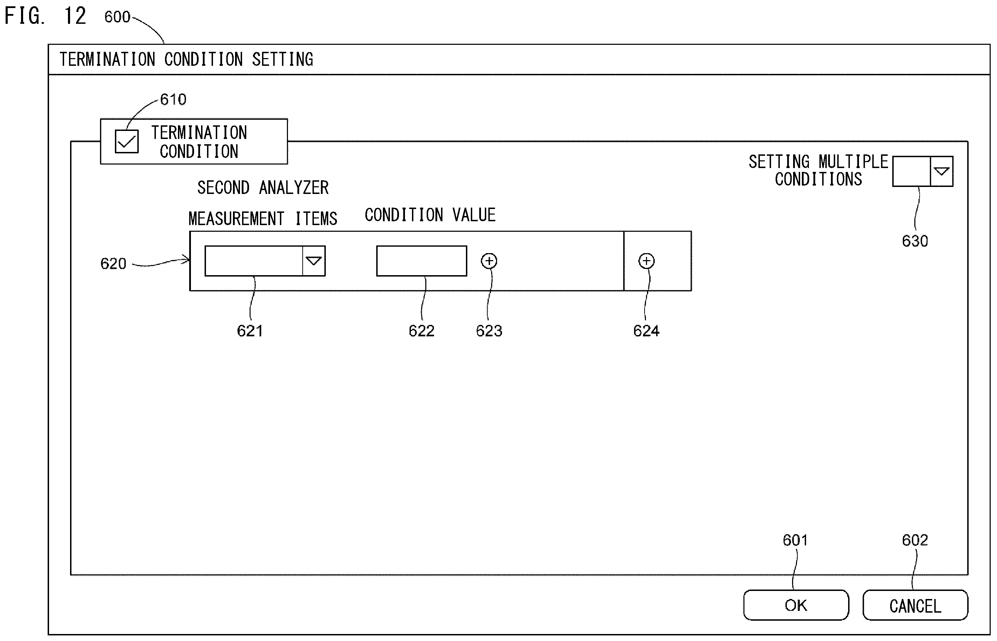

The operator operates a check box 551 to activate or inactivate the condition for terminating multiple measurements at the second analyzer. When the check box 551 is checked, the condition to terminate the multiple measurements becomes active. When the termination condition setting button 552 is operated by the operator, a termination condition setting screen 600 for setting the termination condition (see FIG. 12) appears on the display 43.

When the OK button 501 is operated, the controller 41 of the management device 40 stores the rules set in the rule setting screen 500 and the setting conditions temporarily stored in the termination condition setting screen 600 to be described later in the storage 42 and closes the rule setting screen 500. When the cancel button 502 is operated, the controller 41 discards the rule set in the rule setting screen 500 and closes the rule setting screen 500. When the delete button 503 is operated, the controller 41 deletes the rule set in the rule setting screen 500 from the storage 42.

FIG. 12 is a diagram schematically showing a termination condition setting window 600 for setting the termination condition.

The operator uses checkbox 610 to set whether to activate the conditions for terminating multiple measurements by the second analyzer, i.e., the conditions set in the end condition setting screen 600. When checkbox 610 is checked, the conditions for terminating multiple measurements become active. The state of checkbox 610 is linked to the state of checkbox 551 in the rule setting screen 500.

The operator uses the dropdown menu 621 to select the measurement item of the second analyzer that will determine the termination of the multiple measurements by the second analyzer, and enters the condition value for the measurement item in the text box 622. If there are multiple condition values, the operator can use the add button 623 to display additional text boxes 622. If the operator wants to add more conditions for the multiple measurements, the operator can use the add button 624. This will add more condition areas 620. When multiple condition areas 620 are displayed, the operator can use the dropdown menu 630 to set whether the conditions set in the multiple condition areas 620 should be determined by "and" or "or".

When the OK button 601 is operated, the controller 41 of the management device 40 temporarily stores the termination condition set in the termination condition setting screen 600 in the storage 42 and closes the termination condition setting screen 600. When the cancel button 602 is operated, the controller 41 discards the termination condition set in the termination condition setting screen 600 and closes the termination condition setting screen 600.

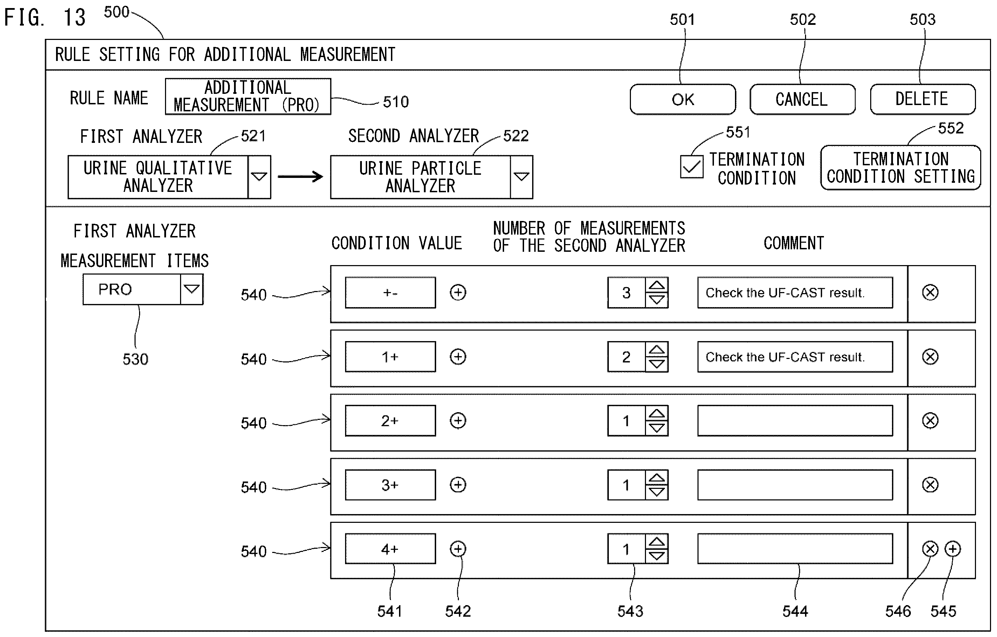

FIG. 13 is a diagram schematically showing the rule setting screen 500 in which the rule of the additional measurement regarding "Protein" as shown in FIG. 7 is set.

As shown in FIG. 7, since five conditions are set in the measurement item "protein (PRO)" of the first analyzer (urine qualitative analyzer 10), five condition areas 540 are displayed. When a plurality of condition areas 540 are displayed, a delete button 546 for deleting each condition area 540 is provided.

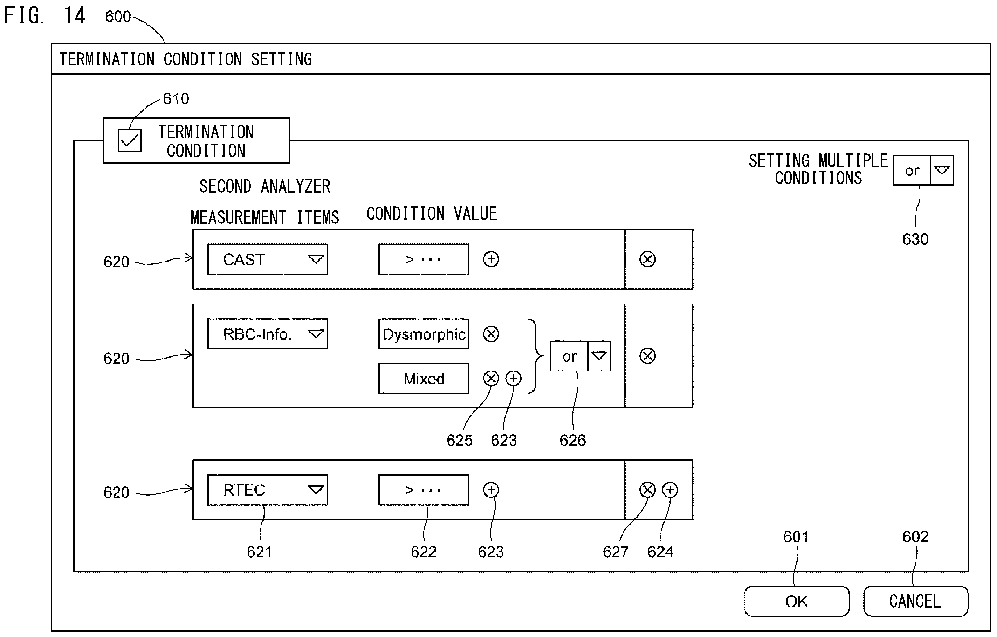

FIG. 14 is a diagram schematically showing the termination condition setting window 600 in which the termination condition regarding "Protein" as shown in FIG. 7 is set.

As shown in FIG. 7, in this case, since the termination condition relating to the measurement items "cast," "red blood cell morphology information (RBC-Info.)," and "tubular epithelial cells (RTEC)" of the second analyzer (urine particle analyzer 20) are set, three conditional areas 620 are displayed. When a plurality of text boxes 622 are displayed for entering the condition values of the second analyzer measurement items, a delete button 625 is provided for deleting each text box 622, and a pull-down menu 626 is provided for setting whether the condition values of each text box 622 are determined by either "and" or "or". Further, when a plurality of condition areas 620 are displayed, the delete button 627 for deleting each condition area 620 is provided.

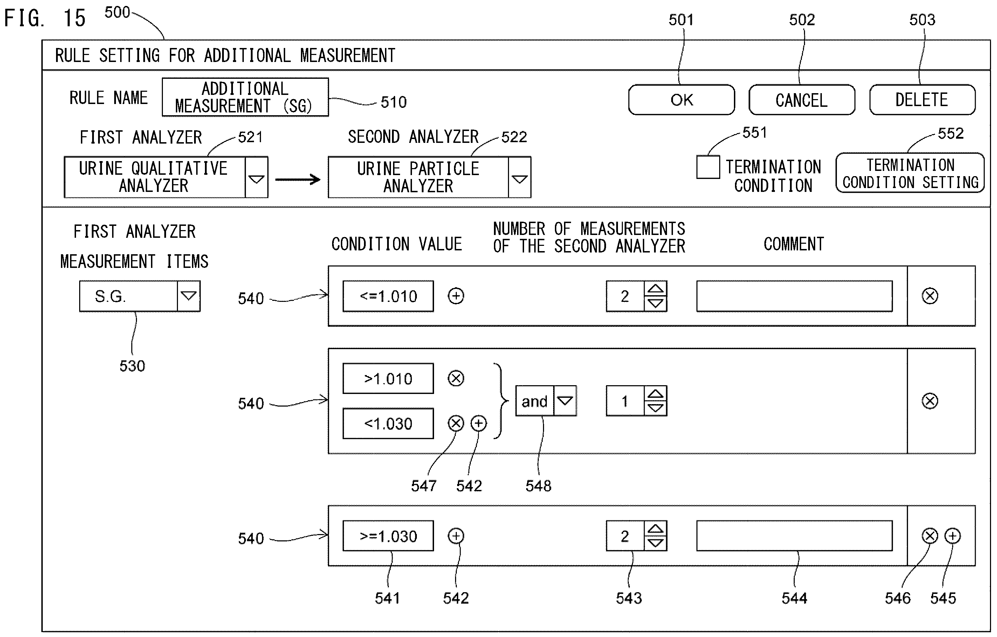

FIG. 15 is a diagram schematically showing the rule setting screen 500 in which the rule of the additional measurement regarding "Specific Gravity" as shown in FIG. 7 is set.

As shown in FIG. 7, since the three conditions are set in the measured item "specific gravity (s.g.)" of first analyzer (urine qualitative analyzer 10), three condition areas 540 are displayed. When a plurality of text boxes 541 for entering the condition values of the first analyzer measurement items are displayed in the condition area 540, a delete button 547 for deleting each text box 541 and a pull-down menu 548 for setting whether to determine the condition values of each text box 541 by "and" or "or" are provided. **In** this instance, as shown in FIG. 7, since there is no termination condition of measuring in the second analyzer (urine particle analyzer 20), the check box 551 is set to the off state.

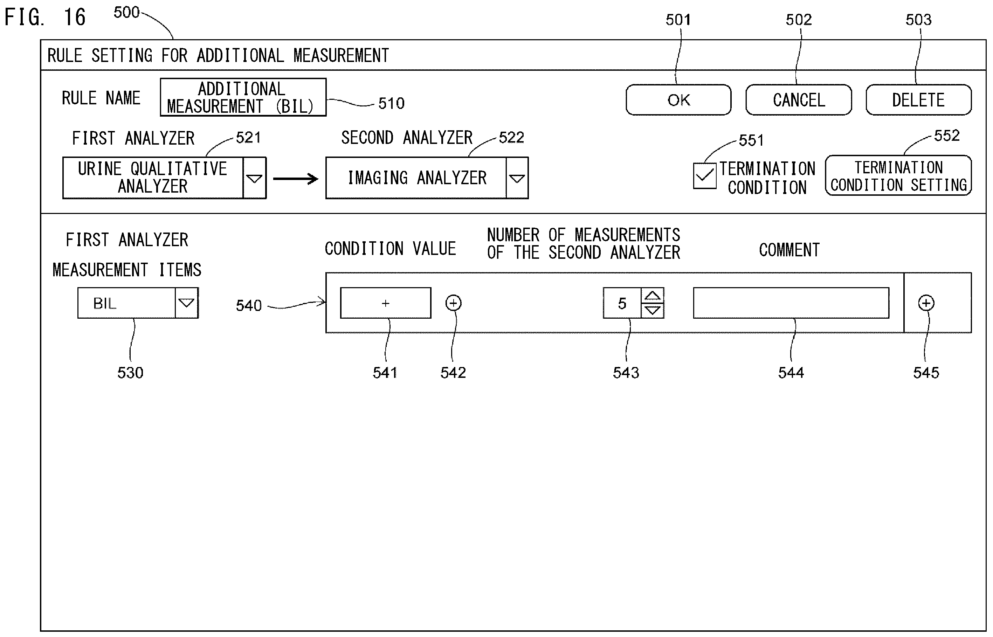

FIG. 16 is a diagram schematically showing the rule setting screen 500 in which the rule of the additional measurement regarding "Bilirubin" as shown in FIG. 8 is set.

As shown in FIG. 8, since one condition is set in the measurement item "bilirubin (BIL)" of the first analyzer (urine qualitative analyzer 10), one condition area 540 is displayed. **In** this case, the number of times measured (imaging times) of the second analyzer (imaging analyzer 30) is set to 5 times.

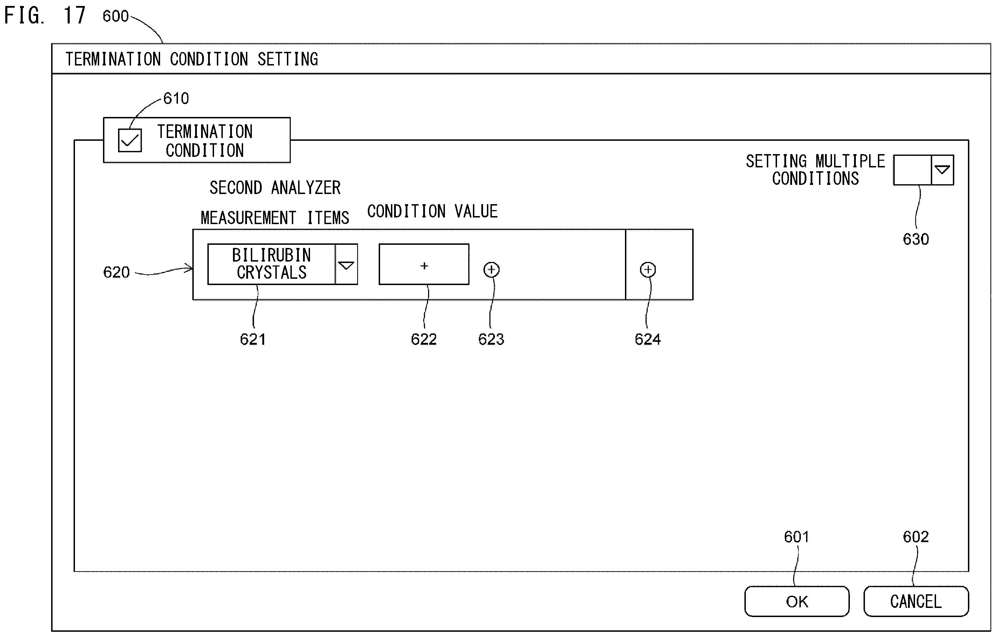

FIG. 17 is a diagram schematically showing a termination condition setting window 600 in which the termination condition regarding "Bilirubin" as shown in FIG. 8 is set.

As shown in FIG. 8, in this case, since one termination condition for the measurement item "bilirubin crystals" of the second analyzer (imaging analyzer 30) is set, one conditional area 620 is displayed. **In** this instance, the text box 622 indicating the conditional value is entered with "+", and it is set as a termination condition that at least one bilirubin crystal is detected in the imaging analyzer 30. Inequality signs or other symbols may be entered in the text box 622, and the termination condition may be set such that the detection of bilirubin crystals equal to or greater than a predetermined value terminates the measurements.

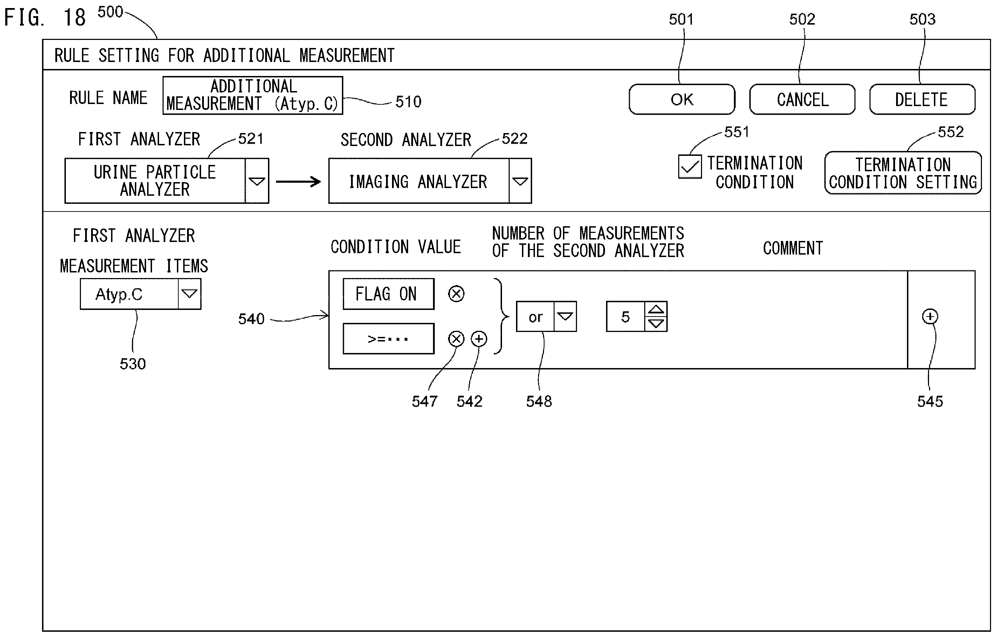

FIG. 18 is a diagram schematically showing the rule setting screen 500 in which the rule of the additional measurement regarding "Atypical Cell" as shown in FIG. 9 is set.

As shown in FIG. 9, since one condition is set for the measurement item "atypical cell (Atyp.c)" of first analyzer (urine particle analyzer 20), one condition area 540 is displayed.

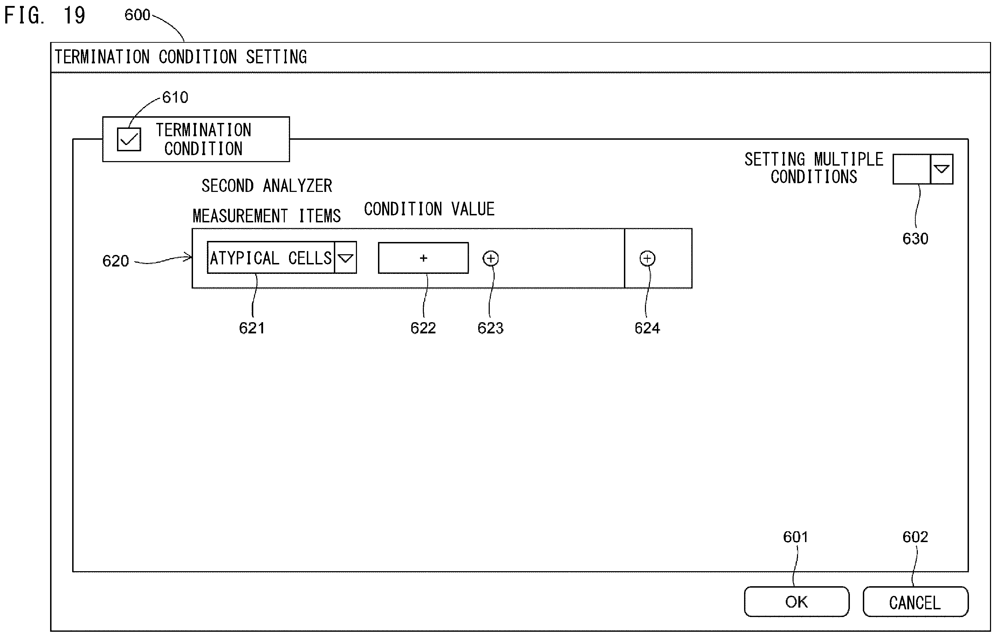

FIG. 19 is a diagram schematically showing a termination condition setting screen 600 in which the termination condition regarding "Atypical Cell" as shown in FIG. 9 is set.

As shown in FIG. 9, in this case, since one termination condition for the measurement item "atypical cell" of second analyzer (imaging analyzer 30) is set, one conditional area 620 is displayed.

Next, a screen for displaying the measurement results will be described.

A result list screen 700 and the detailed result screen 800 shown below are also displayed on the display 43 of the management device 40 by the controller 41 of the management device 40. When the operator of the management device 40 inputs an operation to the screen through the input unit 44, the controller 41 of the management device 40 executes processing in response to the input operation.

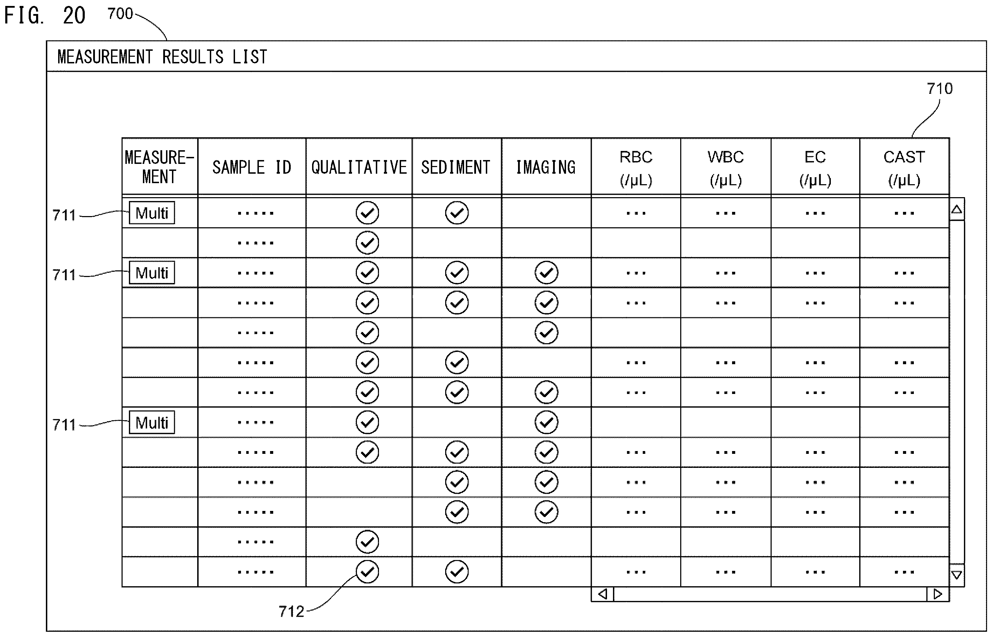

Fig. 20 is a diagram schematically showing the result list screen 700 for displaying a list of measurement results.

A list area 710 shows the measurement results associated with the sample **ID** for each row and includes columns of "Measurement", "Sample ID", "Qualitative", "Sediment", "Imaging", and a plurality of measurement items.

In the "Measurement" column, a mark 711 is displayed if an order to make more than one additional measurement (multiple measurements) is set for the second analyzer (either urine particle analyzer 20 or imaging analyzer 30). The columns "Qualitative", "Sediment", and "Imaging" are marked with a check mark 712 when measured at urine qualitative analyzer 10, urine particle analyzer 20 and imaging analyzer 30, respectively. The columns of the plurality of measurement items indicate the measurement results of all of the measurement items that can be obtained in urine qualitative analyzer 10, urine particle analyzer 20 and imaging analyzer 30.

When referring to the detailed measurement results of the subject sample, the operator operates the corresponding row of the list area 710. Accordingly, the detail result screen 800 (see FIGS. 21 to 23) containing detailed measurement results associated with the sample **ID** of the operated row is displayed on the display 43.

FIG. 21 is a diagram schematically showing the detailed result screen 800 for displaying a detailed measurement result when a tab 821 is operated.

A sample information display area 810 displays the sample **ID,** the date of sample collection, and the name of the subject from whom the sample was collected, etc. Further, the mark 811 is displayed in the sample information display area 810, similarly to the mark 711 shown in FIG. 20, when an order for perform ing a plurality of additional measurements (multiple measurements) is set for the second analyzer (either of the urine particle analyzer 20 and the imaging analyzer 30).

A display area 820, according to the operation of the tab 821 to 824, various measurement results are displayed. The display area 820 of FIG. 21 illustrates a state in which the measurement results are displayed in a list and comments are displayed by manipulating the tab 821.

The operator operates a pull-down menu 831 to determine which one is to be displayed in a list area 832 from the measurement results acquired by the urine qualitative analyzer 10 (urinary qualitative test result), the measurement results acquired by the urine particle analyzer 20 (urine sediment test result), or the measurement results acquired by the imaging analyzer 30 (imaging test result).

When the sample of interest is measured multiple times in the device corresponding to the pull-down menu 831, each measurement result is displayed in columns such as "Result 1" and "Result 2" in the list area 832. The total result value based on each measurement result is displayed in the column of "Result". The overall result value is, for example, the average of the results of each measurement. On the other hand, when the sample of interest is measured only once in the device corresponding to the pull-down menu 831, the measurement result of the one time is displayed in the "Results" in the list area 832, and columns such as "Results 1" and "Results 2" are omitted.

Review comments 833 display comments indicating the fact that the subject sample has been measured multiple times in the device corresponding to the pull-down menu 831 and the measurement items on which multiple measurements have been made. For example, the review comment 833 of FIG. 21 shows that more than one measurement was made in the urine particle analyzer 20 based on the measurement of protein (PRO) in the urine qualitative analyzer 10. The review comment 833 displays the comments entered in the text box 544 (see FIG. 11) of the rule setting screen 500.

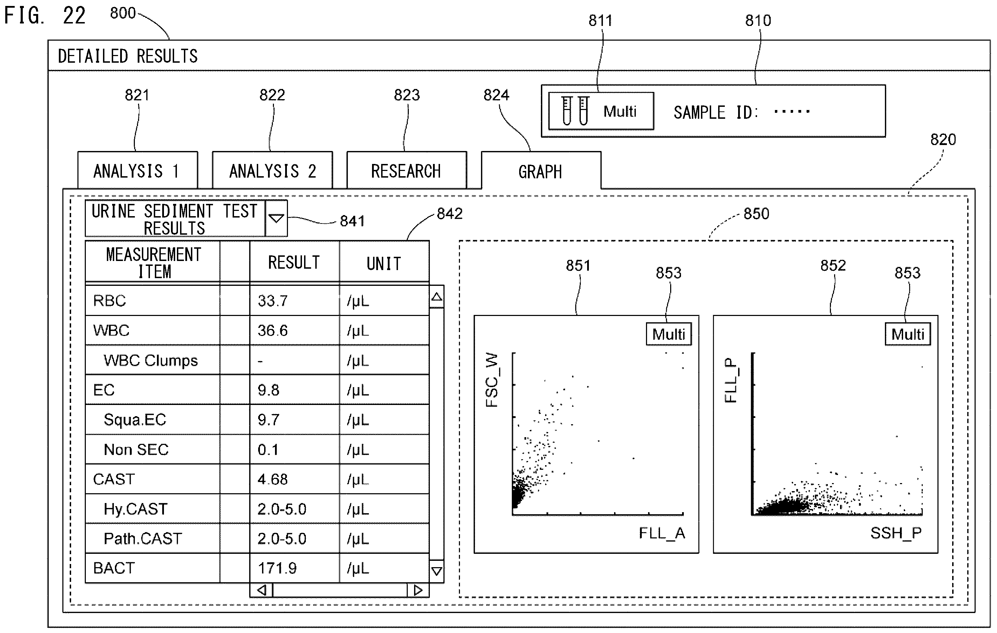

Fig. 22 is a diagram schematically showing the detailed result screen 800 for displaying a detailed measurement result when the tab 824 is operated.

The display area 820 of FIG. 22 illustrates a state in which the measurement results are displayed in a list and a graph such as a scattergram or histogram is displayed by operating the tab 824.

The operator operates a pull-down menu 841 to determine which one is to be displayed in the list area 842 from urinary qualitative test results, urine sediment test results, or imaging test results. Depending on the operation of the pull-down menu 841, one of the graphs acquired by the urine qualitative analyzer 10, the graphs acquired by the urine particle analyzer 20, and the images acquired by the imaging analyzer 30 are displayed in the graph area 850. In the graph area 850 of FIG. 22, two scattergrams 851 and 852 acquired by the urine particle analyzer 20 are displayed, marks 853 indicating that these scattergrams 851 and 852 have been acquired by a plurality of measurements are displayed.

Here, when multiple measurements are performed in one analyzer for one sample, the graph obtained by the analyzer is generated by superimposing the plurality of measurement results. Generally, the urine particle analyzer 20 and imaging analyzer 30 detect a few particles, resulting in fewer particles being plotted on the scattergram in a single run. This may make it difficult to classify the particles using a scattergram. In contrast, according to the embodiment, when multiple measurements are made, the plots obtained in the multiple measurements are superimposed on the graph. This makes it easier to classify the particles using a scattergram because of the large number of particles plotted on the scattergram.

Instead of the scattergram generated by superimposing a plurality of measurement results, the scattergram of each of the original measurement results may be displayed individually.

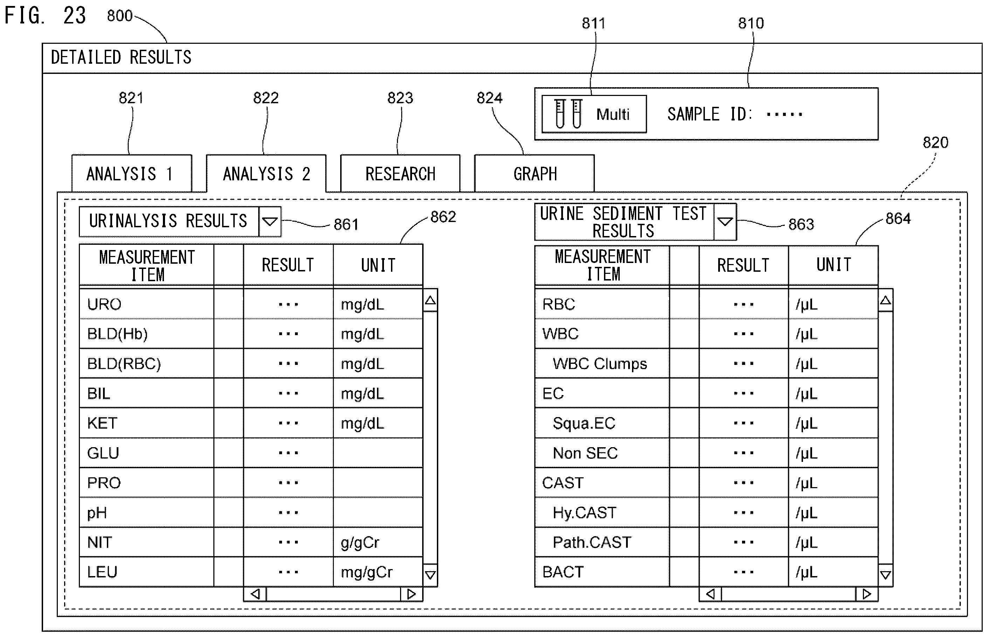

Fig. 23 is a diagram schematically showing the detailed result screen 800 for displaying a detailed measurement result when the tab 822 is operated.

In the display area 820 of FIG. 23, an example is shown where the measurement results obtained by two devices are displayed side by side when tab 822 is selected.

The operator operates a pull-down menu 861 to determine which one is to be displayed in the list area 862 from urinary qualitative test results, urine sediment test results, or imaging test results. The operator operates a pull-down menu 863 to determine which one is to be displayed in the list area 864 from urinary qualitative test results, urine sediment test results, or imaging test results.

When the tab 823 is manipulated, the display area 820 displays the measurement results, for example, the measurement items of the urine particle analyzer 20 "urinary tract epithelial cells (Tran.EC)," "tubular epithelial cells (RTEC)," "small round epithelial cells (SRC)," "atypical cell (Atyp.c)," "red blood cell morphology information (RBC-Info.)" and the like as research items among the measurement items of each device.

Next, the process performed by the management device 40, the first analyzer and the second analyzer will be described with reference to the flowchart.

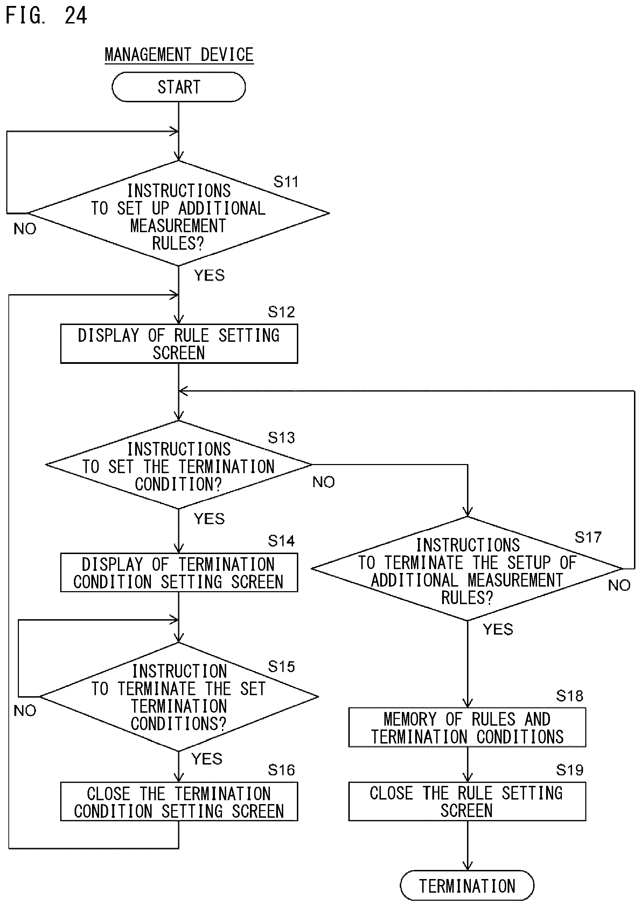

FIG. 24 is a flowchart illustrating a process of setting an additional measurement rule.

When the controller 41 of the management device 40 determines that an instruction to display the rule setting screen 500 has been input via the input unit 44 (step S11: YES), the controller 41 displays the rule setting screen 500 on the display unit 43 in step S12. This allows the operator of the management device 40 to input the rules for additional measurements into the rule setting screen 500 via the input unit 44. When the controller 41 determines that the termination condition setting button 552 has been operated to display the termination condition setting screen 600 in the rule setting screen 500 (step S13: YES), the controller 41 displays the termination condition setting screen 600 on the display unit 43 in step S14. This allows the operator of the management device 40 to input the end conditions into the end condition setting screen 600 via the input unit 44.

When the controller 41 determines that the OK button 601 or the cancel button 602 has been operated in the termination condition setting screen 600 to finish setting of termination conditions (step S15: YES), the controller 41 closes the termination condition setting screen 600 in step S16 and returns the process to step S12. If the OK button 601 is operated in the termination condition setting screen 600, the controller 41 temporarily stores the termination conditions set in the termination condition setting screen 600.

When the controller 41 determines that the OK button 501, the cancel button 502, or the delete button 503 has been operated in the rule setting screen 500 to finish setting the rules for additional measurements (step S17: YES), the controller 41 stores the rules for additional measurements input in the rule setting screen 500 and the termination conditions input in the end condition setting screen 600 in the storage 42 in step S18. If the cancel button 502 is operated in the rule setting screen 500, the controller 41 omits the process in step S18. If the delete button 503 is operated in the rule setting screen 500, the controller 41 deletes the previously stored rules for additional measurements and the termination conditions from the storage 42. Then, in step S19, the controller 41 closes the rule setting screen 500.

Next, the process of the first analyzer, the second analyzer, and the management device 40 when measurements are sequentially performed by the first analyzer and the second analyzer will be described with reference to flowcharts.

As described above, in embodiments, there are three combinations of the first analyzer and second analyzer. Specifically, there are combinations: (1) the first analyzer and second analyzer are urine qualitative analyzer 10 and urine particle analyzer 20, respectively; (2) the first analyzer and second analyzer are urine qualitative analyzer 10 and imaging analyzer 30, respectively; and (3) the first analyzer and second analyzer are urine particle analyzer 20 and imaging analyzer 30, respectively. In combinations (1) to (3), additional measurements are made according to the rules shown in FIGS. 7, 8, and 9, respectively.

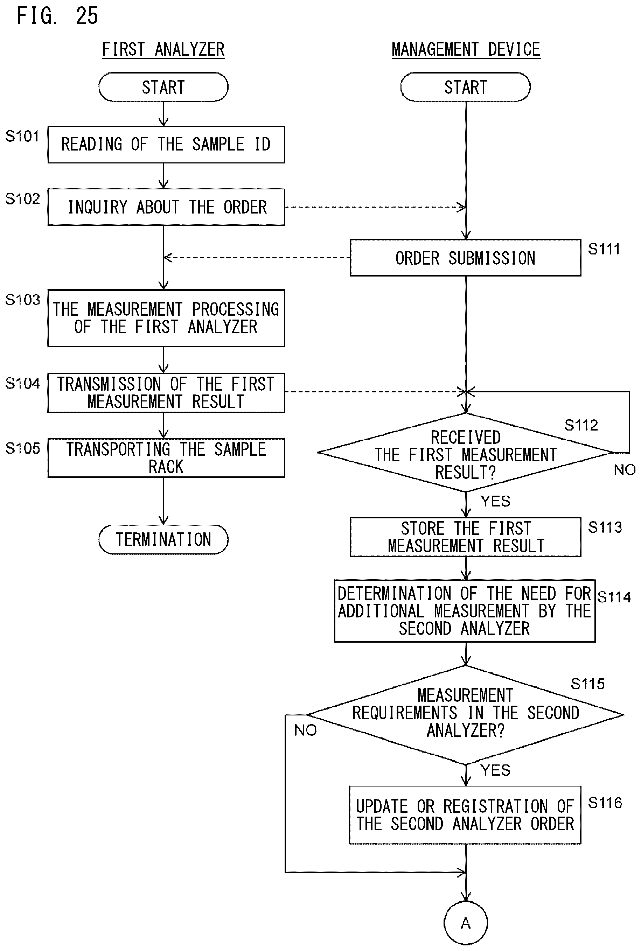

FIG. 25 is a flowchart illustrating the process of the first analyzer and the management device 40.

In step S101, the controller of the first analyzer reads the sample ID from the sample container 101 using the first analyzer reader. In step s102, the controller of the first analyzer transmits the query information of the order including the sample ID read in step S101 to the management device 40. When the controller 41 of the management device 40 receives the query information of the order from the first analyzer, the controller 41 acquires the order of the first analyzer associated with the sample ID included in the query information of the received order from the storage 42 of the management device 40 or an external host computer. Then, in step S111, the controller 41 of the management device 40 transmits the acquired order to the first analyzer.

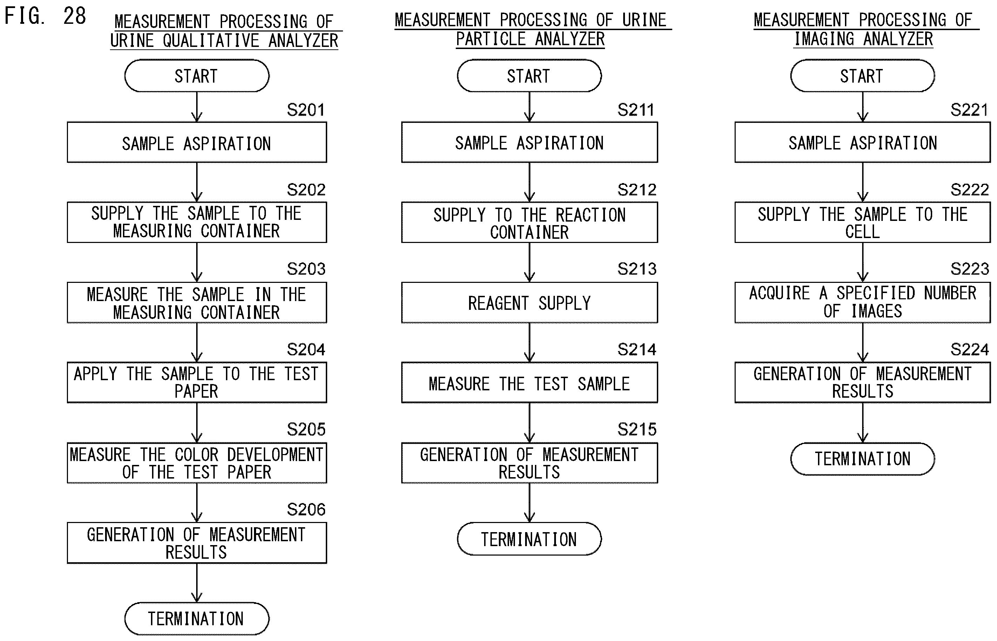

In step S103, the controller of the first analyzer performs a measurement process by the first analyzer based on the order received from the management device 40, obtains the measurement result of the first analyzer (the first measurement result). The measurement process will be described with reference to FIG. 28. In step S104, the controller of the first analyzer transmits the first measurement result obtained in step S103 to the management device 40 in association with the sample ID. Thereafter, in step S105, the controller of the first analyzer conveys the sample rack 100.

When the sample rack 100 holds the unprocessed sample by the first analyzer, the controller of the first analyzer performs the processing of steps S101 to S105 for other samples. When the measurement of all the samples held in the sample rack 100 is completed, the controller of the first analyzer conveys the sample rack 100 from the first analyzer conveying device to the conveying device of the analyzer of the later stage.

When the controller 41 of the management device 40 receives the first measurement result from the first analyzer (step S112: YES), the controller 41 stores the received first measurement result in the storage 42 in step S113. **In** step S114, the controller 41 determines whether additional measurements by the second analyzer are necessary based on the rules for additional measurements for the received first measurement result. **If** the first analyzer is the urine qualitative analyzer 10, the necessity determination is made based on the rules for additional measurements shown in FIGS. 7 and 8. **If** the first analyzer is the urine particle analyze 20, the necessity determination is made based on the rules for additional measurements shown in FIG. 9.

When the controller 41 determines that an additional measurement by the second analyzer is required in step S114 (step S115: YES), the controller 41 updates or registers the order of the second analyzer of the sample of interest in step S116.

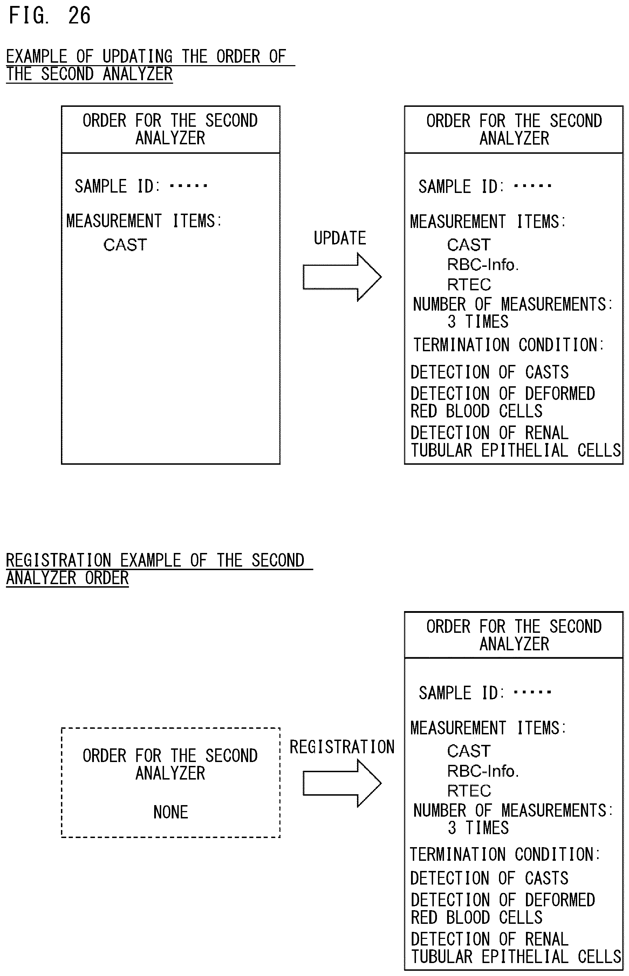

FIG. 26 is a diagram schematically illustrating updating and registering orders of second analyzer.

As shown in the upper left side of FIG. 26, if the order for the second analyzer of the target specimen has been pre-set by the operator or the like, that is, if the original order exists, the controller 41 updates the original order stored in the storage 42 or the host computer with the measurement contents of the second analyzer corresponding to the first measurement result of the first analyzer that meets the conditions for performing additional measurements, as shown in the upper right side of FIG. 26. **In** the upper left side of FIG. 26, the measurement item specified in the original order is "casts (CAST)." **If** the first row of the additional measurement rules in FIG. 7 is applied in this case, the original order is updated with the contents of the additional measurements, as shown in the upper right side of FIG. 26. As a result, the measurement items "red blood cell morphology information (RBC-Info.)" and "renal tubular epithelial cells (RTEC)" are added, the number of measurements is set to three, and the termination conditions are added.

Even if the termination condition is set in the rule for additional measurements, all measurement items of the second analyzer may be set in updating the additional order. Further, if the original order is present as shown in the upper left side of FIG. 26, an additional order shown in the upper right side of FIG. 26 may be registered separately.

On the other hand, as shown in the lower left side of FIG. 26, if there is no original order of the subject sample, as shown in the lower right side of FIG. 26, the controller 41 newly registers the order of the second analyzer based on the rule of additional measurement, and stores it in the storage 42 or the host computer. All measurement items of the second analyzer may be set in the registration of the additional order.

**If** the first measurement result is applicable to a plurality of conditions for performing an additional measurement, for example, the maximum number of times among the numbers of additional measurements corresponding to each condition is set to the number of additional measurements of the order of the second analyzer, and all the termination condition corresponding to each condition is set to the termination condition of the order of the second analyzer.

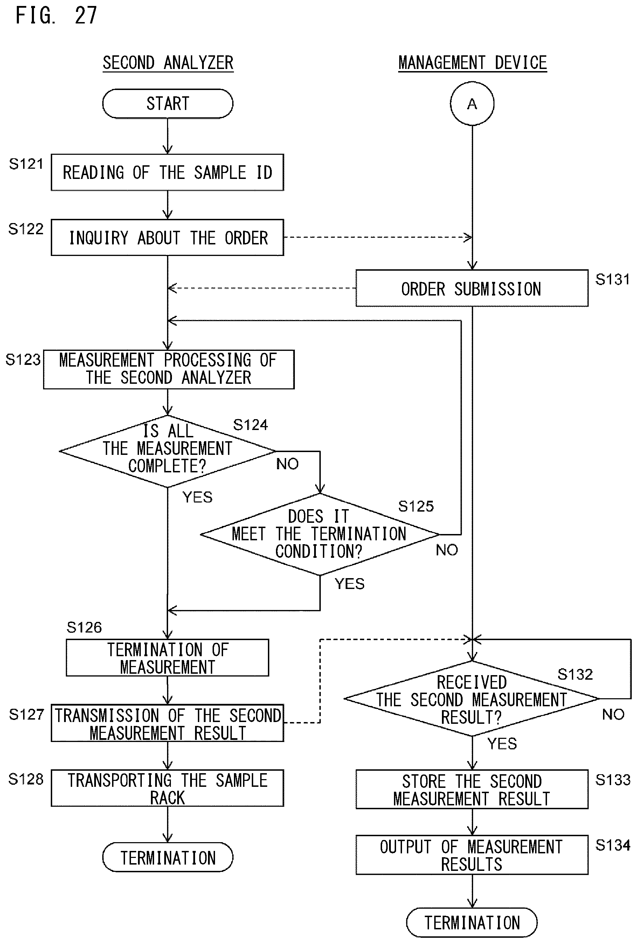

Returning to FIG. 25, when the controller 41 determines that additional measurement by the second analyzer is not required in step S114 (step S115: no), the controller 41 does not perform the process of step S116. If it is determined NO in step S115, and if step S116 is executed, the process proceeds to step S131 of FIG. 27.

FIG. 27 is a flowchart illustrating the process of the second analyzer and the management device 40.

In step S121, the controller of the second analyzer reads the sample ID from the sample container 101 using the second analyzer reader. In step S122, the controller of the second analyzer transmits the query information of the order including the sample ID read in step S121 to the management device 40. When the controller 41 of the management device 40 receives the query information of the order from the second analyzer, the controller 41 acquires the order of the second analyzer associated with the sample ID included in the query information of the received order from the storage 42 of the management device 40 or an external host computer. Then, in step S131, the controller 41 of the management device 40 transmits the acquired order to the second analyzer.

If the order of the second analyzer is not stored for the subject sample, the controller 41 of the management device 40 transmits the data indicating that there is no order of second analyzer to the second analyzer. In this instance, the process of steps S123 to S127 to be described later is omitted in the second analyzer.

In step S123, the controller of the second analyzer performs a measurement process by the second analyzer based on the order received from the management device 40, obtains the measurement result of the second analyzer (second measurement result). By the process of step S123, one measurement by the second analyzer is completed. Subsequently, in step S124, the controller of the second analyzer determines whether or not measurements of the measurement number specified by the order of the second analyzer have been performed on the sample of interest. If the measurements of the number of measurements specified in the order of the second analyzer have been completed (step S124: YES), in step S126, the controller of the second analyzer terminates the measurements by the second analyzer.

If the measurements of the number of measurements specified in the order of the second analyzer have not been completed (step S124: NO), in step S125, the controller of the second analyzer determines whether or not the second measurement result obtained in the measurement of the previous step S123 satisfies the termination condition specified in the order of second analyzer. If the second measurement result satisfies the termination condition (step S125: YES), in step S126, the controller of the second analyzer terminates the measurements by the second analyzer.

If the measurements of the measurement number specified in the order of the second analyzer have not been completed (step S124: NO), and the second measurement result does not satisfy the termination condition (step S125: NO), the controller of the second analyzer returns the process to step S123, and again performs the measurement process by the second analyzer.

As described above, the determination of the termination conditions (step S125) is performed for each measurement process (step S123) of the second analyzer. The measurement termination process (step S126) in response to the termination conditions being met is executed upon completion of the measurement by the second analyzer by which the termination conditions were determined. In other words, the measurement termination process (step S126) when the termination conditions are met is executed at a timing when the measurement by the second analyzer is not being performed. This ensures that the measurement by which the termination conditions were determined is executed to the end.

**In** step S127, the controller of the second analyzertransmits the number of times the measurement process of step S123 is performed and all of the second measurement results acquired in step S123 to the management device 40 in association with the sample ID. Thereafter, in step S128, the controller of the second analyzer conveys the sample rack 100.

Again, if the sample rack 100 holds the unprocessed sample by the second analyzer, the controller of the second analyzer performs the processing of steps S121 to S127 for the other sample. When measurements of all the samples held in the sample rack 100 are completed, if the second analyzer is urine particle analyzer 20, the controller of the second analyzer conveys the sample rack 100 to the transport device 30a of the subsequent imaging analyzer 30, and if the second analyzer is imaging analyzer 30, the controller of the second analyzer stores the sample rack 100 to the left end of the transport device 30a.

When the controller 41 of the management device 40 receives the second measurement result from the second analyzer (step S132: YES), the controller 41 stores the second measurement result received in the storage 42 in step S133. Thereafter, in step S134, the controller 41 outputs a measurement result in response to an instruction of the operator.

**In** the embodiment, the controller 41 outputs the measurement result by displaying the measurement result on the display 43, but not limited thereto, for example, may output the measurement result by transmitting to another device.

FIG. 28 is a flowchart illustrating a process of measuring the urine qualitative analyzer 10, urine particle analyzer 20 and imaging analyzer 30 in order from left to right.

As shown in the flowchart on the left side of FIG. 28, in step S201, the controller 11 of the urine qualitative analyzer 10 controls the dispensing unit 14 so as to agitate the sample in the sample container 101 conveyed by the transport device 10a and so as to aspirate the sample from the sample container 101. The controller 11controls the dispensing unit 14 so as to supply the sample aspirated to the measurement container in step S202. The controller 11 controls the refractive index measuring unit 15 so as to measure the sample in the measurement container in step S203. The controller 11 controls the dispensing unit 14 so as to attach the aspirated sample to the test strip in step S204, and controls the color measuring unit 16 so as to measure the color change of the test strip in step S205.

In step S206, the controller 11 generates, based on the measurement data acquired in step S203, measurement results of the urine qualitative analyzer 10 relating to the measurement items of the specific gravity, the color tone, and the turbidity as shown in FIG. 6. The controller 11 generates, based on the measurement data acquired in step S205, measurement results relating to the other measurement items of the urine qualitative analyzer 10 shown in FIG. 6.

The measurements performed in the urine qualitative analyzer 10 are all performed under the same condition, in other words, in the same sequence. That is, a time during which a sample is agitated in step S201, an amount of a sample to be aspirated in step S201, a position of a tip of an aspiration tube when aspirating the sample in step S201, an amount of a sample to be supplied to a measurement container in step S202, a time from being supplied to the measurement container in step S202 until the measurement is performed in step S203, an amount of a sample to be attached at step S204, a time from being attached in step S204 to being measured in step S205, a wavelength and an intensity of a light in the measurement performed in step S205, etc. are the same among all measurements.

As shown in the central flowchart of FIG. 28, in step S211, the controller 21 of the urine particle analyzer 20 controls the dispensing unit 24 so as to agitate the sample in the sample container 101 conveyed by the transport device 20a and so as to aspirate the sample from the sample container 101. The controller 21, in step S212, controls the dispensing unit 24 so as to supply the aspirated sample to the reaction chamber (chamber), and in step S213, controls the sample preparation unit 25 so as to supply the reagent to the reaction chamber. This prepares the measurement sample to be measured in the reaction chamber. The controller 21, in step S214, controls the optical measurement unit 26 to measure the measurement sample prepared in the reaction chamber.

In step S215, the controller 21, based on the measurement data acquired in step S214, generates a measurement result for the measurement items of the urine particle analyzer 20 shown in FIG. 6.

The measurements performed in the urine particle analyzer 20 are all performed under the same condition, in other words, in the same sequence. That is, a time during which a sample is agitated in step S211, an amount of a sample to be aspirated in step S211, a position of a tip of an aspiration tube when aspirating the sample in step S211, an amount of a sample to be supplied to the reaction chamber in step S212, an amount of a reagent supplied to the reaction chamber in step S213, a reaction time of a sample and a reagent in the reaction chamber, a velocity of a measurement sample flowing the flow cell 205, a wavelength and an intensity of a light in the measurement performed in step S214, etc. are the same among all measurements.

If the urine particle analyzer 20 is second analyzer, each measurement process of the second analyzer performed in step S123 of FIG. 27 is performed under the same conditions as described above, according to the central flowchart of FIG. 28.

As shown in the flowchart on the right side of FIG. 28, in step S221, the controller 31 of the imaging analyzer 30 controls the dispensing unit 34 so as to agitate the sample in the sample container 101 conveyed by the transport device 30a and so as to aspirate the sample from the sample container 101. The controller 31, in step S222, controls the dispensing unit 24 to supply the sample aspirated to the cell 321 (see FIG. 5). In step S223, the controller 31 controls the imaging unit 35 so as to capture the sample supplied to the cell and so as to acquire a predetermined number of images.

In step S224, the controller 31 generates, based on the measurement data (images) acquired in step S223, a measurement result for the measurement items of the imaging analyzer 30 shown in FIG. 6.

The measurements performed in the imaging analyzer 30 are all performed under the same conditions, in other words, in the same sequence. That is, a time during which a sample is agitated in step S221, an amount of a sample to be aspirated in step S221, a position of a tip of an aspiration tube when aspirating the sample in step S221, an amount of a sample to be supplied to the cell 321 in step S222, a time from a sample is supplied to the cell 321 until starting the Imaging (time to precipitate the particle), a time for emitting the light source 301, an amount of light of the light source 301, a transfer rate of the cell 321, a capturing setting and a number of images of the image sensor 312 are the same among all measurements.

If the imaging analyzer 30 is second analyzer, each measurement process of the second analyzer performed in step S123 of FIG. 27 is performed according to the flow chart on the right side of FIG. 28, in the same manner as described above.

The measurement processes of the urine qualitative analyzer 10, the urine particle analyzer 20, and the imaging analyzer 30 shown in FIG. 28 start with the agitating and aspiration of the sample and complete by the generation of the measurement results. Therefore, each measurement performed on the basis of a pre-set order in each analyzer starts with the agitating and the aspiration of the sample and complete by the generation of the measurement results. Each measurement performed on the basis of an additional order in the second analyzer also starts with the agitating and the aspiration of the sample and complete by the generation of the measurement results.

### <Effect of sample analysis method and sample analysis system according to an embodiment>

In the above embodiment, there are three combinations of the first analyzer and the second analyzer. Specifically, there are the following combinations: (1) the first analyzer and the second analyzer are the urine qualitative analyzer 10 and the urine particle analyzer 20, respectively; (2) the first analyzer and the second analyzer are the urine qualitative analyzer 10 and the imaging analyzer 30, respectively; and (3) the first analyzer and the second analyzer are the urine particle analyzer 20 and the imaging analyzer 30, respectively. In any of combinations (1) to (3), the measurement methods of the first analyzer and the measurement methods of the second analyzer are different from each other.

As shown in FIG. 25, the sample analysis method of the embodiment includes a step of measuring the sample by the first analyzer (step S103) and a step of determining the measurement conditions by the second analyzer (steps S114 to S116) based on the first measurement result that varies depending on the amount of analyte in the sample.

When the first analyzer is the urine qualitative analyzer 10, as described with reference to FIGS. 7 and 8, "+-", "1+", "2+", "3+", "4+", etc. which are the measurement results of the measurement item "protein (PRO)", the measurement values (numerical values) of the measurement items "specific gravity (S.G.)" and "pH", and "OTHER", "L YELLOW", "STRAW", "YELLOW", "AMBER", "RED", "DK BROWN", etc. which are the measurement results of the measurement item "color", are the first measurements that vary depending on the quantity of analyte in the sample. When the first analyzer is the urine particle analyzer 20, as described with reference to FIG. 9, the measurement values (numerical values) of the measurement items "atypical cells (Atyp.c)", "lipid droplet", "cast", "epithelial cells (EC)" and "sperm" are the first measurement results which vary depending on the quantity of analyte in the sample.

According to this method, since the measurement of the second analyzer in the measurement conditions based on the first measurement result that varies according to the amount of analyte in the sample is performed, in the second analyzer, the appropriate measurement according to the condition of the sample can be performed, and it can be avoided that excessive measurement is performed. Therefore, it is possible to provide reliable analysis results efficiently.

As shown in FIG. 25, the step of determining the measurement conditions includes a step of determining whether or not the necessity of a plurality of measurements by the second analyzer based on the first measurement result (step s114).

This method allows to obtain reliable analysis results in the second analyzer because more than one measurement is performed in the second analyzer as needed.

As shown in FIGS. 25 and 27, the sample analysis method of the embodiment includes a step to terminate the measurements by the second analyzer (step S126), wherein the step includes terminating the measurements by the second analyzer based on that the second measurement result obtained satisfies a predetermined termination condition (step S125: YES) during the measurement period (step S123 to S125) by the second analyzer in accordance with the determination step(step S114) .

According to this method, in addition to the measurements performed at the first analyzer, the subsequent second analyzer performs the measurement, thereby providing highly reliable analysis results. Further, in the measurement at the second analyzer of the later stage, the measurement of the second analyzer is terminated on the basis that the second measurement result satisfies the predetermined termination condition. This saves reagents consumed by the second analyzer, samples supplied to the second analyzer, times required to measure samples, and so on.

As shown in FIG. 27, a judgement of the termination condition (step S125) is performed for each measurement by the second analyzer (step S123). The termination of the measurements by second analyzer (step S126) is executed in accordance with the end of measurement, whose measurement result satisfies the termination condition, by the second analyzer.

According to this method, the measurement of the second analyzer for which the judgement of the termination condition is performed can be reliably performed to the end, and the measurement of the second analyzer after the second measurement result is determined to satisfy the termination condition is not started. Thus, it is possible to reliably obtain the second measurement result from the determination of the termination condition is performed, and the reagent consumed by the second analyzer, the sample supplied to the second analyzer, time-consuming to measure the sample and the like can be saved.

As shown in FIGS. 7-9, the termination condition for terminating the measurement by the second analyzer includes obtaining the second measurement results estimated by the first measurement result used to determine whether or not to perform the test by the second analyzer.

This method saves reagents consumed by the second analyzer, samples supplied to the second analyzer, times required for measurement of the sample, etc. when the second measurement result can be acquired while increasing the possibility of obtaining the second measurement result of the second analyzer estimated by the first measurement result of the first analyzer by a plurality of measurements by the second analyzer. Further, if the second measurement result can be obtained, for the subject collected sample, for example, it can be determined that the possibility of contracting a disease or the like assumed from the first measurement result is extremely high.

As shown in FIG. 25, when it is determined that the measurement by the second analyzer is required based on the first measurement result of the first analyzer (step S115: YES), the step of generating an order defining the measurement by second analyzer (step S116) is executed.

This approach generates an order defining the measurement by the second analyzer when the second analyzer measurements are required, so that the second analyzer can perform measurements smoothly based on the generated order.

As shown in FIG. 27, when the second measurement result obtained during the measurement period of the second analyzer performed according to the order (steps S123 to S125) satisfies the predetermined termination condition (steps S125: YES), the measurements by the second analyzer ends (steps S126).

According to this method, even during the measurement period according to the order, when the second measurement result satisfies the predetermined termination condition, the measurements by the second analyzer is terminated. This saves reagents, samples, time, etc. as well as obtaining the necessary second measurement results.

As shown in FIG. 27, when the second measurement result obtained during the measurement period of the second analyzer performed according to the order (steps S123 to S125) does not satisfy the predetermined termination condition (steps S125: NO), when the measurement number of the second analyzer reaches the measurement number specified by the order (steps S124: YES), the measurements by the second analyzer ends (step S126).

According to this method, even if the termination condition is not satisfied, the measurement by the second analyzer ends when the number of measurements of the second analyzer reaches the number of measurements specified by the order. Therefore, it is possible to smoothly terminate the determination of the second analyzer.

As shown in FIG. 26, the order of the second analyzer includes the number of measurements.

According to this configuration, the second analyzer can measure smoothly according to the order.

**In** addition to the number of measurements, the order of the second analyzer may include the measurement items, the amount of sample aspirated, the amount of sample to be measured, and the measurement duration. Again, the second analyzer can measure smoothly according to the order.

**If** more than one measurement number are included in the order of the second analyzer, more than one measurement by the second analyzer are performed under the same condition.

According to this method, in the second analyzer, it is possible to prevent the deviation of the second measurement result of the second analyzer due to different measurement conditions. Further, by performing the measurements under the same condition, it is possible to suppress complicated control in the second analyzer.

If the second analyzer order contains more than one measurement number, the sample is aspirated from the sample container 101 containing the sample for each of the multiple measurements by the second analyzer.

When the measurement is performed by aspirating the sample at each time in a plurality of measurements as described above, the following advantages are obtained, for example, in comparison with the case where the measurement is performed by aspirating the amount of the sample required for multiple measurements at once. That is, because multiple measurements are performed as separate measurements, it is easier to terminate multiple measurements at the end of a given measurement. This shortens the time required for measurement and reduces the amount of sample consumption. Since the amount of sample used for measurement is always the same, it is not necessary to set multiple measurement modes in order to measure different sample quantities, thus simplifying the control of measurement. Further, since it is sufficient to store the quantity of the sample used for one measurement in the analyzer, it is possible to reduce the capacity of the container such as the measurement vessel, the reaction chamber and the cell. Thus, the device can be miniaturized.

If the second analyzer order contains more than one number of measurements, the sample is agitated prior to aspiration of the sample for each of the multiple measurements by the second analyzer.

If agitating of the sample is not performed prior to aspiration of the sample, particle may precipitate in the sample contained in the sample container 101 and the aspirated sample may not contain enough particle. In contrast, when the sample is agitated prior to aspiration of the sample as described above, the sample sufficiently containing particle is aspirated, so that a sufficient number of particle can be detected in the device that aspirates the sample. Also, when multiple measurements are performed in the second analyzer, it is possible to increase the likelihood of detecting the particle of interest. Furthermore, when the termination condition is set, the possibility of obtaining the measurement result of the object can be increased with fewer measurement times, so that the time required for measurement can be shortened, and the sample quantity used for measurement can be reduced.

The order of the second analyzer is generated based on the first measurement result of the first analyzer when it is determined that the measurement by the second analyzer is required.

According to this method, for example, as shown in FIG. 7, in accordance with the first measurement results of the first analyzer measurement item "protein (PRO)", the measurement number of each second analyzer is determined, the order of the second analyzer is generated based on the measurement number of the determined second analyzer. That is, the order of the second analyzer is generated based on the first measurement which is the basis for determining that the measurement by the second analyzer is required. Thus, it is possible to appropriately generate the order of the second analyzer according to the first measured result of the first analyzer.

The sample analysis method of the embodiment further includes a condition for determining the necessity of measurement by the second analyzer, the content of an order defining the measurement by the second analyzer generated when the measurement by the second analyzer is determined to be required based on the first measurement result, and a step of setting a predetermined termination condition for terminating the measurements by the second analyzer (steps S12 to S18 in FIG. 24).

According to this approach, the operator can perform the various settings described above for the second analyzer according to the operation of the facilities, etc.

As shown in FIGS. 7-9, in the step of setting the above, the conditions for determining the necessity, the content of the order, and the predetermined termination condition are set for each measuring item of the first analyzer.

In this way, detailed settings can be made for each first analyzer measure.

In the step of determining whether it is necessary (steps S114 in FIG. 25), based on the first measurement result, the necessity of measurement by at least one of a plurality of types of second analyzer is determined. For example, as shown in FIG. 7, when the first measurement result of the measurement item "protein (PRO)" is "+-", it is determined that measurement by the urine particle analyzer 20 is required. As shown in FIG. 8, when the first measurement result of the measurement item "COLOR" is "OTHER", it is determined that measurement by the imaging analyzer 30 is required.

According to this method, when a plurality of types of second analyzer is provided, together with the determination of the necessity of measurement, it is possible to determine the second analyzer of the measurement is required among the plurality of types of second analyzer. Therefore, the required measurements can be reliably performed in the appropriate type of second analyzer.

The sample to be measured is a urine sample.

The second analyzer is frequently performed on urinary samples because not only the measurement results based on the first analyzer but also the measurement results based on the second analyzer are often required . Consequently, saving of reagents, samples, times, etc. in such the second analyzer is particularly useful for urine samples.

The first analyzer is the urine qualitative analyzer 10 and the second analyzer is the urine particle analyzer 20 using a flow cytometer, or the imaging analyzer 30.

According to this configuration, detailed measurements can be performed at the urine particle analyzer 20 or the imaging analyzer 30 (second analyzer) in urine qualitative analyzer 10 (first analyzer) with a large number of urine samples.

If the first analyzer is the urine qualitative analyzer 10, the second analyzer is the urine particle analyzer 20 using a flow cytometer, and the measurements by the second analyzer are performed more than once, the measurements of more than one time of the second analyzer are performed under the same condition. Measurement condition in the urine particle analyzer 20 relates to, for example, the amount of sample to be aspirated in step S211, the amount of sample to be supplied to the reaction chamber in step S212, the amount of reagent to be supplied to the reaction chamber in step S213, the reaction time of the sample and the reagent in the reaction chamber, the velocity of the measurement sample flowing in the flow cell 205, the wavelength and intensity of the light when the measurement is performed in step S214, etc.

According to this method, in the urine particle analyzer 20 (second analyzer), it is possible to prevent the deviation of the second measurement result due to different measurement conditions. Further, by performing the measurement under the same condition, it is possible to suppress the complicated control in the urine particle analyzer 20 (second analyzer).

If the first analyzer is the urine qualitative analyzer 10, the second analyzer is the imaging analyzer 30, and the second analyzer is measured more than once, a predetermined number of images are captured for the sample-filled cell 321 in the second analyzer more than one measurement. The capturing condition in the imaging analyzer 30, relates to, for example, the amount of the sample to be aspirated in step S221, the amount of the sample to be supplied to the cell 321 in step S222, the time from the sample is supplied to the cell 321 to start the imaging (time to precipitate the particle), the time for emitting the light source 301, the amount of light of the light source 301, the transfer rate of the cell 321, the capturing setting and the number of images pickup of the image sensor 312 and the like.

According to this approach, in the imaging analyzer 30 (second analyzer), it is possible to prevent the deviation, which is caused by different capturing conditions, for each second measured result generated based on the image. Further, by performing the capturing under the same condition, it is possible to suppress the complicated control in the imaging analyzer 30 (second analyzer).

The first analyzer is the urine particle analyzer 20 using a flow cytometer and the second analyzer is the imaging analyzer 30.

According to this configuration, in the urine particle analyzer 20 (first analyzer), while performing detailed measurements, in the imaging analyzer 30 (second analyzer), it is possible to perform more detailed measurements.

Sample analysis method of the embodiment further comprises a step of outputting the first measurement result and the second measurement result (step S134 of FIG. 27). Thus, for example, the result list screen 700 and the detailed result screen 800 shown in FIGS. 20 to 23 are displayed on the display 43 of the management device 40.

According to this method, the operator can smoothly confirm the first and second measurement results.

As shown in FIG. 1, the sample analysis system 1 includes a plurality of analyzer in which the measurement methods differ from each other, and a management device 40 that receives the measurement results obtained by a plurality of analyzer. As shown in FIG. 25, the management device 40 determines the measurement conditions by the second analyzer (steps S114 to S116) based on the first measurement results of the first analyzer that varies according to the amount of analyte in the sample, and generates an order defining the measurement by the second analyzer (steps S116).

According to this configuration, since the measurement of the second analyzer in the measurement conditions based on the first measurement result that varies according to the amount of analyte in the sample is performed, in the second analyzer, the appropriate measurement according to the circumstances of the sample can be performed, and it can be avoided that excessive measurement is performed. Therefore, it is possible to provide reliable analysis results efficiently.

### <Modification example 1>

In the above embodiment, the determination whether the second measurement result satisfies a predetermined termination condition (steps S125 in FIG. 27) was performed by the second analyzer, but not limited thereto, it may be performed by the management device 40.

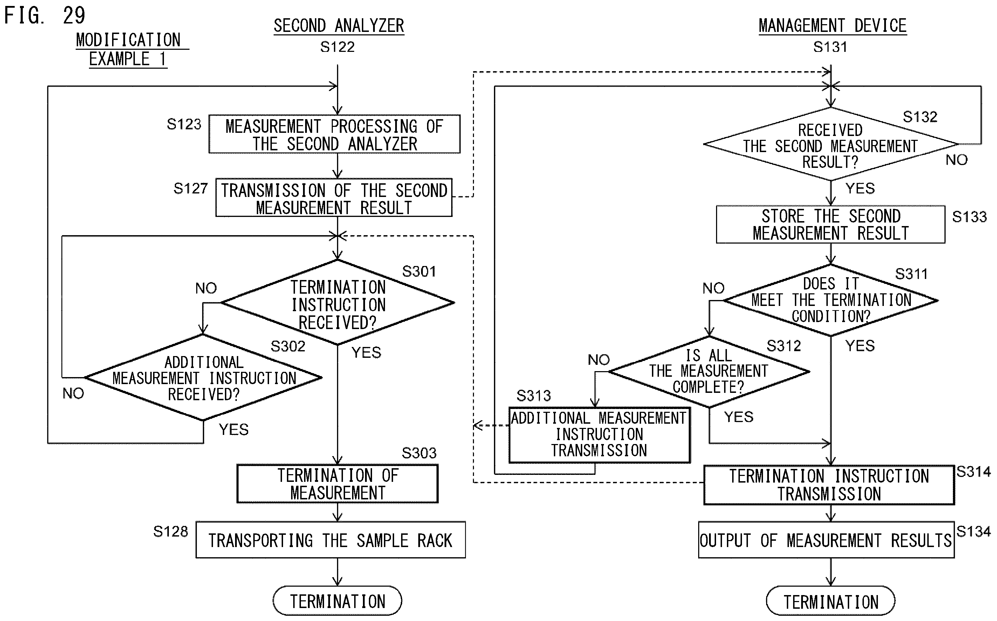

Fig. 29 is a flowchart illustrating the process of the second analyzer and the management device 40 according to the Modification example 1.

In the second analyzer of the modification example 1, in comparison with the embodiment shown in FIG. 27, steps S124 to S126 are omitted, and steps S301 to S303 are added between steps S127 and S128. In the processing of the management device 40 of the modification example 1, as compared with the embodiment shown in FIG. 27, steps S311 to S314 are added between the step S133 and the step S134. In FIG. 29, for convenience, the same steps as in FIG. 27 are partially omitted.

In Modification example 1, the management device 40 manages and controls the number of times measured and the termination condition in the second analyzer as shown below. Accordingly, in the Modification example 1, the order sent from the controller 40 to the second analyzer may not include the number of measurements and termination condition as shown in Fig. 26.

The controller of the second analyzer transmits the second measurement result to the management device 40 in step S127, after the second measurement result is generated by performing the measurement process in step S123. Thereafter, the controller of the second analyzer waits for the process to receive a termination instruction or additional measurement instruction from the management device 40.

When the controller 41 of the management device 40 receives the second measurement result from the second analyzer (step S132: YES), the controller 41 stores the second measurement result in the storage 42 in step S133. Subsequently, in step S311, the controller 41 determines whether or not the second measurement result received in the previous step S132 satisfies the termination condition specified by the order of the second analyzer for the sample of interest. Further, in step S312, the controller 41 determines whether the measurements of the measurement number specified by the order of the second analyzer about the sample of interest has been performed.

If the second measurement result does not satisfy the termination condition specified by the order and the measurement of the measurement number specified by the order is not completed (step S311: NO, step S312: NO), in step S313, the controller 41 transmits the additional measurement instruction to the second analyzer. The controller 41 returns the process to step S132, and waits for the process to receive the second measurement result again. On the other hand, if the second measurement result satisfies the termination condition specified by the order (step S311: YES), or if the measurements of the measurement number specified by the order is completed (step S312: YES), in step S314, the controller 41 transmits the termination instruction to the second analyzer.

The controller of the second analyzer, when receiving the termination instruction from the management device 40 (step S301: YES), in step S303, terminates the measurements by the second analyzer. On the other hand, the controller of the second analyzer, when receiving an additional measurement instruction from the management device 40 (step S301: NO, step S302: YES), returns the process to step S123, and again performs the measurement process by the second analyzer.

In Modification example 1, judgement of the termination condition (step S311) is performed for each measurement process of second analyzer (step S123). the termination of the measurements when satisfying the termination condition (step S303) is executed in accordance with that the measurement, whose measurement result satisfies the termination condition, by the second analyzer is completed. That is, the termination of the measurements when satisfying the termination condition (step S303) is executed at a timing in which the measurement by the second analyzer is not performed. This ensures that the measurement, whose measurement result satisfies the termination condition, can be performed to the end.

According to the Modification example 1, as shown in FIG. 29, the management device 40 judges (step S311) whether any one of the second measurement results obtained during the measurement period by the second analyzer (step S123, S127, S301, S302) satisfies a predetermined termination condition, and based on that the second measurement result satisfies the termination condition (step S311: YES), terminates the measurements by the second analyzer (step S314, S303). Thus, the same effect as in the above embodiment is achieved. That is, since the measurements of the second analyzer are terminated on the basis that any one of the second measurement results satisfies the predetermined termination condition, the reagent consumed in the second analyzer, the sample supplied to the second analyzer, time-consuming to measure the sample and the like can be saved.

### <Modification example 2>

In the above embodiment, the termination of the measurements when satisfying the termination condition is executed at a timing where the measurement by the second analyzer is not performed. However, the embodiments are not limited thereto, the termination of the measurements when satisfying the termination condition may be executed during the measurement operation by the second analyzer.

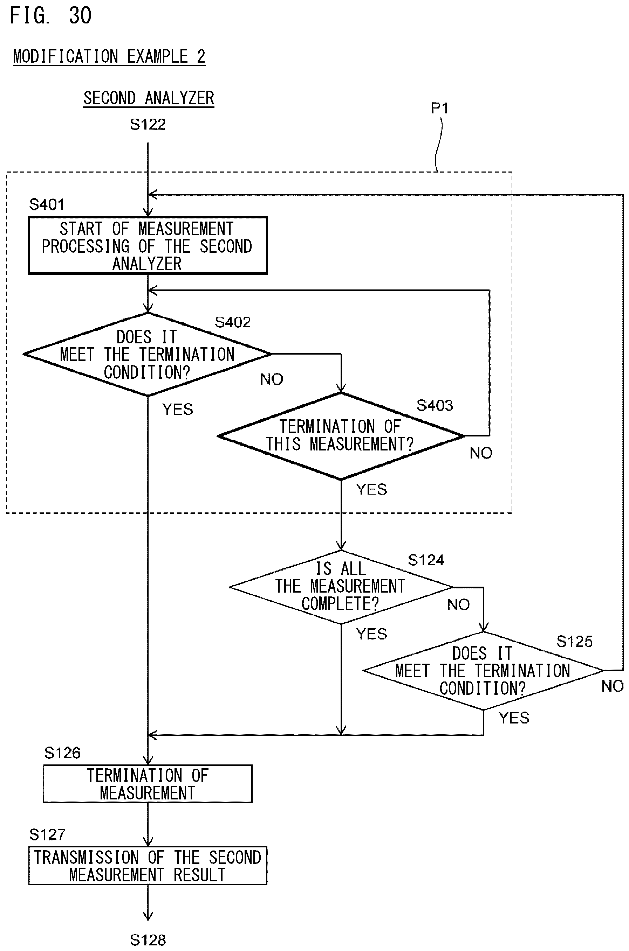

FIG. 30 is a flowchart illustrating a process of second analyzer according to a Modification example 2.

The second analyzer of the Modification example 2 is the imaging analyzer 30. In the process of the second analyzer of the modification example 2, in place of step S123, the steps S401 to S403 are added in comparison with the embodiment shown in FIG. 27. In FIG. 30, for convenience, the same steps as in FIG. 27 are partially omitted.

The controller of the second analyzer starts the measuring process in step S401. Operation period P1 of the measurement started in step S401 is from step S401 to S403. The measurement started in step S401 is continued during the operation period P1. When the measurement is started in step S401, the measurement process of the imaging analyzer 30 shown in the flow chart of the right side of FIG. 28 is started. A predetermined number of images (e.g., 40 images) are sequentially acquired.

In the Modification example 2, unlike the above embodiment, the controller of the second analyzer, while analyzing the image acquired by the measurement process started in step S401, sequentially, generates second measurement results. Then, in step S402, the controller of the second analyzer, during the measurement operation started in step S401, determines whether obtained measurement result satisfies the termination condition.

If the measurement results satisfies the termination condition (step S402: YES), in step S126, the controller of the second analyzer stops the measurement in operation, and terminates the measurement by the second analyzer. That is, the termination process of step S126 based on the determination of step S402 is executed in response to the determination that the termination condition is satisfied during the measuring operation. On the other hand, if the measurement result does not satisfy the termination condition (step S402: NO), in step S403, the controller of the second analyzer determines whether the measurement (current measurement) started in step S401 is completed. If the current measurement is not completed (step S403: NO), the controller of the second analyzer returns the process to step S402, judges the termination condition until the current measurement is completed.

If the current measurement is completed (step S403: YES), the controller of the second analyzer, as in the embodiment, performs the process of steps S124 to S126. That is, if the measurement of the measurement number specified by the order of the second analyzer is completed (step S124: YES), or if the second measurement result of this time satisfies the termination condition (step S125: YES), in step S126, the controller of the second analyzer terminates the measurement by the second analyzer. On the other hand, if the measurement of the number of measurements specified in the order of the second analyzer has not been completed and the second measurement result of this time does not satisfy the termination condition (step S124: NO, step S125: NO), the controller of the second analyzer returns the process to step S401 and starts the next measurement.

According to the Modification example 2, as shown in FIG. 30, the second analyzer determines whether or not the second measurement result obtained during the measurement period by the second analyzer (steps S401 to S403 , S124, S125) satisfies a predetermined termination condition (steps S402, S125), and terminates the measurements by the second analyzer (step S126) based on that the second measurement result satisfies the termination condition (steps S402: YES, steps S125: YES),. Thus, the same effect as in the above embodiment is achieved. That is, since the measurements of the second analyzer are terminated on the basis that the second measurement result satisfies the predetermined termination condition, the reagent consumed in the second analyzer, the sample supplied to the second analyzer, time-consuming to measure the sample and the like can be saved.

Further, according to the Modification example 2, the judgement of the termination condition in step S402 is performed during the measurement operation (operation period P1) for each measurement of the second analyzer. Further, the termination process of step S126 is executed in response to a determination that the second measurement result satisfies the termination condition (step S402: YES) . According to this method, when the second measurement result is determined to satisfy the termination condition, even in the middle of the measurement by the second analyzer, the measurements by the second analyzer is terminated. This saves time-consuming for analyte determination in the second analyzer.

### <Modification example 3>

In the above-described Modification example 1, the termination of the measurements when satisfying the termination condition is executed at a timing where the measurement by the second analyzer is not performed. However, the embodiments are not limited thereto, the termination of the measurements when satisfying the termination condition may be executed during the measurement operation by the second analyzer.

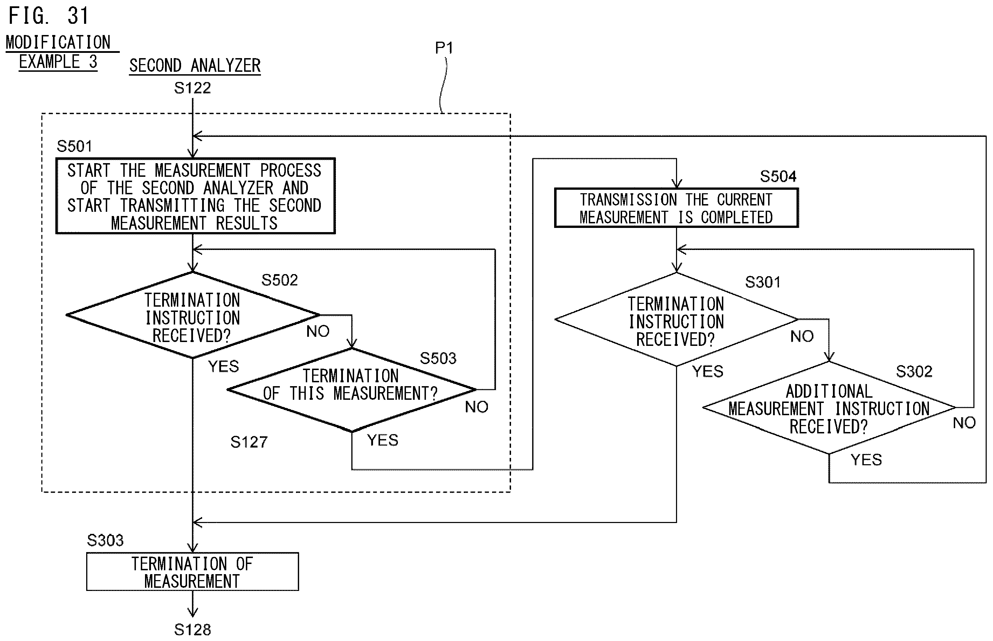

FIG. 31 is a flowchart illustrating a process of second analyzer according to Modification example 3.

The second analyzer of Modification example 3 is an imaging analyzer 30. In the process of the second analyzer of the Modification example 3, in place of steps S123 and S127, steps S501 to S504 are added in comparison with the Modification example 1 shown in FIG. 29.

In step S501, the controller of the second analyzer starts the measuring process. Operation period P1 of the measurement started in step S501 is a step S501 to S503, in the operation period P1, the measurement started in step S501 is continued. When the measurement is started in step S501, the measurement process of the imaging analyzer 30 shown in the flow chart of the right side of FIG. 28 is started, a predetermined number of images (e.g., 40 images) are sequentially acquired.

Further, in step S501, the controller of the second analyzer, while analyzing the image acquired by the measurement process started, sequentially generates the second measurement results, during the measurement operation started, and sequentially transmits obtained second measurement results to the management device 40. As shown in FIG. 29, the management device 40 transmits a termination instruction when the second measurement result received satisfies the termination condition (step S311: YES in FIG. 29).

The controller of the second analyzer, when receiving the termination instruction from the management device 40 (step S502: YES), in step S303, stops the measurement in operation, and terminates the measurements by the second analyzer. That is, the termination process in this case is executed in response to the determination that the termination condition is satisfied during the measuring operation. On the other hand, the controller of the second analyzer, if it has not received the termination instruction from the management device 40 (step S502: NO), in step S503, determines whether the measurement (current measurement) started in step S501 is completed. If the current measurement is not completed (step S503: NO), the controller of the second analyzer returns the process to step S502, and continues to determine the termination instruction reception until the current measurement is completed.

If the current measurement is completed (step S503: YES), in step S504, the controller of the second analyzer transmits to the management device 40 that the current measurement is completed. Thus, the management device 40 increases a count value of the measurement performed in the second analyzer by 1, in step S312 of FIG. 29, and determines whether or not all of the measurements are completed using the count value. Thereafter, the controller of the second analyzer, in the same manner as the Modification example 1 shown in FIG. 29, performs the process of steps S301 to S303.

According to the Modification example 3, as shown in FIGS. 29 and 31, the management device 40 determines whether the second measurement result obtained during the measurement period by the second analyzer (steps S501 to S504 , S301 S302) satisfies a predetermined termination condition (step S311), and terminates the measurements by the second analyzer (steps S314, S303) based on that the second measurement result is satisfied the termination condition (step S311: YES). Thus, the same effect as in the above embodiment is achieved. That is, since the measurements of the second analyzer is terminated on the basis that the second measurement result satisfies the predetermined termination condition, the reagent consumed in the second analyzer, the sample supplied to the second analyzer, time-consuming to measure the sample and the like can be saved.

Further, according to the Modification example 3, the judgement of the termination condition in step S502 is performed during the measurement operation (operation period p1) for each measurement of the second analyzer. Further, the termination process of step S303 is executed in response to a determination that the second measurement result satisfies the termination condition (step S502: YES). According to this method, when the second measurement result is determined to satisfy the termination condition, even in the middle of the measurement by the second analyzer, the measurements by the second analyzer is terminated. This saves time-consuming for analyte analysis in the second analyzer.

### <Examples of other changes>

In the above embodiment, the sample analysis system 1 was a system for analyzing a urine sample, but not limited thereto, it may be a system for analyzing other samples other than a urine sample. For example, the sample analysis system 1 may be a system for analyzing blood samples. The sample analysis system 1 includes, in addition to the management device 40 similar to the embodiment described above, a blood cell analyzer, a imaging analyzer, and a general purpose flow cytometer analyzer arranged in order, for example, from upstream to downstream. The blood cell analyzer measures blood samples prepared from blood samples based on flow cytometry and counts blood cells in blood samples. The imaging analyzer is configured similarly to the imaging analyzer 30, and captures the blood cells in the blood sample. The general purpose flow cytometer analyzer measures blood samples prepared from blood samples based on flow cytometry and counts blood cells in blood samples.

Also in this case, based on the first measurement result obtained by the upstream first analyzer (blood cell analyzer, imaging analyzer), the necessity of a plurality of times of measurements at the downstream second analyzer (imaging analyzer, general purpose flow cytometer analyzer) is determined. Further, the measurements by the second analyzer (imaging analyzer, general purpose flow cytometer analyzer) is terminated depending on the second measurement result, obtained by the second analyzer (imaging analyzer, general purpose flow cytometer analyzer) during the measurement period, satisfies the predetermined termination condition. In this case, the same rules for additional measurement as in Fig. 9 are predetermined, and the necessity of additional measurement is determined based on the rules for additional measurement, and an order is generated.

For example, the management device 40 determines the necessity of additional measurement by the second analyzer (imaging analyzer) based on the measurement result of the measurement item "Blast" obtained by measuring the first analyzer (blood cell analyzer). If the measurement result of the measurement item "Blast" is greater than or equal to a predetermined value, the controller 40 generates an order specifying the number of measurements by the imaging analyzer and the termination condition, etc. The termination condition includes, for example, that the measurement result of "Blasts" by the imaging analyzer is greater than or equal to a predetermined value.

Further, for example, the management device 40, based on a predetermined measurement result obtained by measuring the first analyzer (blood cell analyzer, imaging analyzer), determines the necessity of additional measurement by the second analyzer (general purpose flow cytometer analyzer). When the management device 40 determines that additional measurement is required, it generates an order specifying the number of measurements and termination condition by the general purpose flow cytometer analyzer. The termination condition includes, for example, a measurement of blood cells expressing a predetermined surface antigen is at least a predetermined value. Thus, based on the measurement results of the blood cells expressing a predetermined surface antigen, it is possible to identify a disease such as leukemia.

In this modified example, similarly to the embodiment, since the measurement of the second analyzer in the measurement conditions based on the first measurement result that varies according to the amount of analyte in the sample is performed, in the second analyzer, the appropriate measurement according to the circumstances of the sample can be performed, and it can be avoided that excessive measurement is performed. Therefore, it is possible to provide reliable analysis results efficiently. In addition to measuring with first analyzer (blood cell analyzer and imaging analyzer), the following second analyzer (imaging analyzer, general purpose flow cytometer analyzer) can provide highly reliable analytical results. Further, in the measurement at the second analyzer of the later stage, the measurements of the second analyzer is terminated on the basis that the second measurement result satisfies the predetermined termination condition. This saves reagents consumed by the second analyzer, samples supplied to the second analyzer, times required to measure samples, and so on.

In the above embodiment, the measured results of the urine qualitative analyzer 10, the urine particle analyzer 20, and the imaging analyzer 30 are stored in the management device 40 of the sample analysis system 1, but not limited thereto, and may be stored in an external host computer. In this case, for example, the sample analysis system 1 includes a display terminal for displaying the measurement results and the like, and the display terminal communicates with the host computer to display the measurement results on the display of the display terminal according to the operation of the operator.

In the above embodiment, the urine qualitative analyzer 10 is disposed most upstream, not limited thereto, the urine particle analyzer 20 or imaging analyzer 30 is disposed most upstream, the urine qualitative analyzer 10 may operate as a second analyzer.

In the above embodiment, the order of the second analyzer is generated according to the measurement results of one first analyzer based on the rule of additional measurement, but depending on the measurement results of the two upstream first analyzer, the order of the downstream second analyzer may be generated.

For example, if the measurement result of the urine qualitative analyzer 10 measurement item "occult blood (BLD(Hb))" or "occult blood (BLD(RBC))" is positive (+) and the measurement result of the urine particle analyzer 20 measurement item "red blood cell morphology information (RBC-Info.)" is deformed red blood cells (Dysmorphic?) or homogeneous and mixed red blood cells (Mixed?), an additional order is generated by the imaging analyzer 30. The order in this case is, for example, to perform more than one measurement (multiple measurements) by imaging analyzer 30 and, as a termination condition, to have a positive flag or a threshold or higher measurement result of the measurement item "atypical cells" of the imaging analyzer 30. As the order in this case, it may be set that a plurality of additional measurements (multiple measurements) are performed by the imaging analyzer 30, and that the measurement result of the measurement item "cast" of the imaging analyzer 30 as a termination condition is a predetermined value or more.

In the above embodiment, one condition for performing additional measurement by second analyzer includes only one measurement item of the first analyzer, not limited thereto, may include a plurality of measurement items of the first analyzer. For example, additional measurements may be set when one condition includes the measurement items "protein" and "specific gravity" shown in FIG. 7 and the measurement results of both measurement items apply to the corresponding conditions.

In the above embodiment, if the first analyzer is the urine particle analyzer 20, the terms under which additional measurements are performed by the second analyzer may include a plurality of criteria. For example, it may include a plurality of criteria for determining whether the condition for the measurement result (measurement value) of the measurement item "atypical cells (Atyp.c)" of the urine particle analyzer 20 is included in any of a plurality of numerical ranges. In this case, depending on which numerical ranges the first measurement result of the urine particle analyzer 20 is included in, the measurement conditions by the second analyzer is determined. Thus, in the same manner as in the above-described embodiment, it is possible to perform proper measurement according to the condition of the sample in the second analyzer, and it is possible to avoid excessive measurement. Therefore, it is possible to provide reliable analysis results efficiently.

In the above-described embodiment, the rule of additional measurement is set in the rule setting screen 500 and the termination condition setting screen 600 shown in FIGS. 11 to 19, but not limited thereto, the setting is input by the operator in the program formation, and the controller 41 of the management device 40 may generate an additional order based on this input.

In the above embodiment, if the number of measurements included in the order of the second analyzer (urine particle analyzer 20 and imaging analyzer 30) is a plurality, the measurement of a plurality of times by the second analyzer was performed under the same conditions, not limited thereto, may be different conditions from each other.

Specifically, in the above embodiment, although the positions of the tips of the aspiration tubes when the sample is aspirated from the sample container 101 in each measurement of the multiple measurements were identical to each other, they may be different from each other. For example, when the sedimentation rate of particle in a sample varies with the type of particle, the position of the tip of the aspiration tube (height and horizontal position) at the time of aspiration of the sample in each measurement can be made different from each other to increase the likelihood that the particle in the sample will be sufficiently aspirated in multiple measurements.

Similarly, in the above embodiment, the measurement times may be different from each other in each of the multiple measurements, and the amounts of samples aspirated in each of the multiple measurements may be different from each other. For example, if the measurement result of the measurement item "protein (PRO)" of the urine qualitative analyzer 10 is "+-", the measurement time may be set longer in each of the multiple measurements performed in the urine particle analyzer 20, and the sample volume to be aspirated may be set larger.

In the above embodiment, even if the measurement result of the second analyzer satisfies the termination condition, the measurement of the second analyzer may not be terminated, and measurements of the second analyzer by the number of measurements indicated by the order may be performed. In this case, for example, for each condition area 540 of the rule setting screen 500 shown in FIG. 11, a check box for temporarily disabling the condition is provided. If this check box is set to On, the termination condition is ignored and the second analyzer may measure the number of times the order indicates.

Supplementary explanation will be given with respect to the urine qualitative analyzer 10. The test strip changes its color according to an amount of an analyte the urine qualitative analyzer 10 is to measure. The urine qualitative analyzer 10 measures the color of the test strip to which the sample is spotted by a color sensor and obtains a measurement result indicated by three or more levels according to the amount of the analyte based on the color of the test strip. The three or more levels consist of a first level indicating that the amount of the analyte is a normal level (e.g., denoted by "-"), and a plurality of second levels indicating that the amount of the analyte is higher than the first level (e.g., denoted by "+-," "1+," "2+," "3+," and "4+" as described above). Among the plurality of second levels, the lowest level (e.g., "+-") indicates the lowest amount of the analyte, and the highest level (e.g., "4+") indicates the highest amount of the analyte.

The type of analyte the urine qualitative analyzer 10 is intended to measure corresponds to the measurement items described, for example, with reference to FIG. 6. For example, if the measurement item is occult blood (BLD(Hb)), the analyte is hemoglobin, and the levels representing a measurement result consist of, for example, a first level ("-"), and a plurality of second levels ("+-", "1+", "2+", "3+"). For example, if the measurement item is a protein (PRO), the analyte is a protein, and the levels representing a measurement result consist of, for example, a first level ("-"), and a plurality of second levels ("+-", "1+", "2+", "3+", "4+"). For example, if the measurement item is a leukocyte (LEU), the analyte is a leukocyte, and the levels representing a measurement result consist of, for example, a first level ("-"), and a plurality of second levels ("1+", "2+", "3+"). In the above-mentioned measurement items, when the measurement result is the first level, there is no analyte in the sample, or it is a trace even if it exists.

As described with reference to FIG. 7, in the rule of additional measurement, for example, when the analyte to be measured by the urine qualitative analyzer 10 is a protein, and when a measurement result of the urine qualitative analyzer 10 is "+-", "1+", "2+", "3+", and "4+", the number of measurements of the urine particle analyzer 20 is configurable to be 3, 2, 1, and 1 times, respectively. That is, a number of measurements by the urine particle analyzer 20 when a measurement result of the urine qualitative analyzer 10 is the lowest level ("+-") among the plurality of second levels is configurable to be greater than a number of measurements when the measurement result is the highest level ("4+") among the plurality of second levels. The configuration of the number of measurements may be set, for example, via the rule setting screen 500 described in FIG. 11. Similarly, if the analyte the urine qualitative analyzer 10 is to measure is another analyte such as hemoglobin, leukocyte, etc., the number of measurements by the urine particle analyzer 20 when the measurement result of the urine qualitative analyzer 10 is the lowest level among the plurality of second levels is configurable to be greater than the number of measurements when the measurement result is the highest level among the plurality of second levels.

In the above-described embodiment, the number of measurements of the urine particle analyzer 20 when the measurement result of the urine qualitative analyzer 10 is the lowest level among the plurality of second levels is configurable to be greater than the number of measurements when the measurement result is the highest level among the plurality of second levels, but instead of setting the number of measurements, a measurement time of the sample by the urine particle analyzer 20 may be configurable. That is, the management device 40, based on the measurement result of the urine qualitative analyzer 10, may determine an amount (total amount) of the sample used for the measurement by the urine particle analyzer 20. As the amount of the sample, the number of measurements and the measurement time may be determined. As the measurement time, a time the measurement sample flows through the flow cell 205 of the urine particle analyzer 20 may be used.

The type of analyte the urine particle analyzer 20 is to measure may be determined depending on the type of analyte the urine qualitative analyzer 10 is to measure. For example, the analyte the urine qualitative analyzer 10 is to measure and the analyte the urine particle analyzer 20 is to measure may be the same as each other. For example, the analyte the urine qualitative analyzer 10 is to measure is a leukocyte, and the management device 40 may determine the amount of urine sample used for measurement by the urine particle analyzer 20 depending on the level representing the measurement result by the urine qualitative analyzer 10, and the urine particle analyzer 20 may measure the determined amount of urine sample and count the leukocyte.

The analyte the urine qualitative analyzer 10 to measure and the analyte the urine particle analyzer 20 to measure may be different from each other. For example, the analyte the urine qualitative analyzer 10 to measure is protein, and the management device 40 may determine the amount of urine sample to be used for measurement by the urine particle analyzer 20 depending on the level representing the measurement result by the urine qualitative analyzer 10, and the urine particle analyzer 20 may measure the determined amount of urine sample and count at least one of the casts and tubular epithelial cells. **In** this instance, the urine particle analyzer 20 may determine a presence or absence of deformed red blood cells. Also, for example, the analyte the urine qualitative analyzer 10 is to measure is hemoglobin, and the management device 40 may determine the amount of urine sample to be used for measurement by the urine particle analyzer 20 depending on the level representing the measurement result by the urine qualitative analyzer 10, and the urine particle analyzer 20 may measure the determined amount of the urine sample and count the red blood cell.

**In** the above embodiment, the urine particle analyzer 20 is a device for counting particles in a urine sample using a flow cytometer. However, the present invention is not limited to this, and the urine particle analyzer 20 may be a device for capturing urine samples that flow through or are stored in a cell and counting particles in the urine sample based on the captured images. **In** this instance, the imaging analyzer 30 may be omitted from the sample analysis system 1. **In** this case, the amount of the urine sample used for capturing may be determined as the amount of the sample.

**In** the above embodiment, the management device 40 is provided separately from the urine qualitative analyzer 10 and the urine particle analyzer 20 and the like. However, the present invention is not limited thereto, and the management device 40 may be incorporated into the urine qualitative analyzer 10 or the urine particle analyzer 20. In this case, the controller 41, the storage 42, the display 43, and the input unit 44 may share the functions with the management device 40, the urine qualitative analyzer 10 or the urine particle analyzer 20.

Embodiments of the present invention can be varied as appropriate within the scope of the technical ideas set forth in the appended claims.

## Claims

1. A sample analysis system (1), comprising:
a plurality of analyzers including a first analyzer (10, 20) and a second analyzer (20, 30), wherein the first analyzer and the second analyzer employ different measurement methods, the first analyzer being configured to measure a urine sample using a test strip that changes its color according to an amount of an analyte in the urine sample and obtain, based on the color of the test strip, a first measurement result representing the amount of the analyte in three or more levels, and wherein the three or more levels consist of (i) a first level indicating that the amount of the analyte is a normal level, and (ii) a plurality of second levels indicating that the amount of the analyte is higher than the first level; and
a management device (40) configured to obtain the first measurement result, and determine a sample amount to be measured by the second analyzer based on the level represented by the first measurement result,
wherein the sample amount corresponding to the lowest level of the plurality of second levels is configurable to be greater than the sample amount corresponding to the highest level of the plurality of second levels, and
wherein the second analyzer is configured to measure the urine sample having the sample amount determined by the management device and obtain a second measurement result of the urine sample.

2. The sample analysis system according to claim 1, wherein the first analyzer is a urine qualitative analyzer, and the second analyzer is a urine particle analyzer.

3. The sample analysis system according to claim 1, wherein the management device obtains the second measurement result and outputs the first measurement result and the second measurement result in association with identification information of the urine sample.

4. The sample analysis system according to claim 1, wherein the management device determines, as the sample amount to be measured, a number of measurements performed on the urine sample by the second analyzer.

5. The sample analysis system according to claim 4, wherein the second analyzer obtains, in multiple measurements, the second measurement result based on a plurality of measurement results of the urine sample obtained from multiple measurements.

6. The sample analysis system according to claim 4, wherein the second analyzer aspirates, in each of the multiple measurements, the urine sample from a sample container and measures the aspirated urine sample.

7. The sample analysis system according to claim 6, wherein the second analyzer cancels, in response to a predetermined termination condition being satisfied before the second analyzer performs a last aspiration of multiple aspirations, an aspiration and a measurement to be performed after the predetermined termination condition is satisfied.

8. The sample analysis system according to claim 7, wherein the predetermined termination condition is that at least one of the measurement results obtained by each measurement by the second analyzer satisfies a predetermined detection condition.

9. The sample analysis system according to claim 7, wherein the predetermined termination condition is that a predetermined analyte is detected.

10. The sample analysis system according to claim 7, wherein the management device receives a setting of the predetermined termination condition.

11. The sample analysis system according to claim 6, wherein the second analyzer agitates the urine sample in the sample container before each aspiration of multiple measurements.

12. The sample analysis system according to claim 6, wherein the second analyzer performs each of multiple measurements according to same sequence.

13. The sample analysis system according to claim 6, wherein the second analyzer aspirates same amount of the urine sample among the multiple measurements.

14. The sample analysis system according to claim 1, further comprising a transport device configured to transport a sample container accommodating the urine sample to the first analyzer and the second analyzer.

15. The sample analysis system according to claim 1, wherein both the first measurement result and the second measurement result are results regarding the same analyte from each other.

16. The sample analysis system according to claim 15, wherein the analyte is a leukocyte.

17. The sample analysis system according to claim 1, wherein the first measurement result is a result regarding an amount of a first analyte as the analyte, and the second measurement result is a result regarding an amount of a second analyte different from the first analyte.

18. The sample analysis system according to claim 17, wherein the first analyte is a protein, and the second analyte is at least one of a cast, a deformed red blood cell, and a renal tubular epithelial cell.

19. The sample analysis system according to claim 17, wherein the first analyte is hemoglobin, and the second analyte is a red blood cell.

20. The sample analysis system according to claim 1, wherein the second analyzer is a urine particle analyzer, and the second analyzer measures the urine sample in a period of time corresponding to the sample amount determined by the management device.

21. A sample analysis method using a plurality of analyzers including a first analyzer (10, 20) and a second analyzer (20, 30),
wherein the first analyzer and the second analyzer employ different measurement methods, comprising:
measuring, by the first analyzer, a urine sample using a test strip that changes its color according to an amount of an analyte in the urine sample;
obtaining, by the first analyzer, a first measurement result indicating the amount of the analyte in three or more levels based on the color of the test strip,
wherein the levels consist of (i) a first level indicating that the amount of the analyte is a normal level, and (ii) a plurality of second levels indicating the amount of the analyte is higher than the first level;
obtaining the first measurement result by a management device (40); determining, by the management device, a sample amount to be measured by the second analyzer based on the level of the amount of the analyte indicated by the first measurement result,
wherein the sample amount corresponding to the lowest level of the plurality of second levels is configurable to be greater than the sample amount corresponding to the highest level of the plurality of second levels; and
by the second analyzer, measuring the urine sample having the sample amount determined by the management device and obtaining a second measurement result of the urine sample.

## Patentansprüche

1. Probenanalysesystem (1), umfassend:
eine Vielzahl von Analysatoren, einschließlich eines ersten Analysators (10, 20) und eines zweiten Analysators (20, 30), wobei der erste Analysator und der zweite Analysator unterschiedliche Messverfahren verwenden, wobei der erste Analysator so konfiguriert ist, dass er eine Urinprobe unter Verwendung eines Teststreifens misst, der seine Farbe entsprechend einer Menge eines Analyten in der Urinprobe ändert, und basierend auf der Farbe des Teststreifens ein erstes Messergebnis erhält, das die Menge des Analyten in drei oder mehr Niveaus darstellt, und wobei die drei oder mehr Niveaus aus (i) einem ersten Niveau bestehen, das anzeigt, dass die Menge des Analyten ein normales Niveau ist, und (ii) einer Vielzahl von zweiten Niveaus bestehen, die anzeigen, dass die Menge des Analyten höher als das erste Niveau ist; und
eine Verwaltungsvorrichtung (40), die dazu konfiguriert ist, das erste Messergebnis zu erhalten, und eine Probenmenge zu bestimmen, die vom zweiten Analysator basierend auf das durch das erste Messergebnis dargestellte Niveau gemessen werden soll,
wobei die Probenmenge, die dem niedrigsten Niveau der Vielzahl von zweiten Niveaus entspricht, so konfiguriert werden kann, dass sie größer als die Probenmenge ist, die dem höchsten Niveau der Vielzahl von zweiten Niveaus entspricht, und
wobei der zweite Analysator dazu konfiguriert ist, die Urinprobe mit der durch die Verwaltungsvorrichtung bestimmten Probenmenge zu messen und ein zweites Messergebnis der Urinprobe zu erhalten.

2. Probenanalysesystem nach Anspruch 1, wobei der erste Analysator ein qualitativer Urinanalysator ist, und der zweite Analysator ein Urinpartikelanalysator ist.

3. Probenanalysesystem nach Anspruch 1, wobei die Verwaltungsvorrichtung das zweite Messergebnis erhält und das erste Messergebnis und das zweite Messergebnis in Verbindung mit Identifikationsinformationen der Urinprobe ausgibt.

4. Probenanalysesystem nach Anspruch 1, wobei die Verwaltungsvorrichtung als zu messende Probenmenge eine Anzahl von Messungen bestimmt, die an der Urinprobe durch den zweiten Analysator durchgeführt wird.

5. Probenanalysesystem nach Anspruch 4, wobei der zweite Analysator in mehreren Messungen das zweite Messergebnis basierend auf einer Vielzahl von Messergebnissen der Urinprobe erhält, die aus mehreren Messungen erhalten wurden.

6. Probenanalysesystem nach Anspruch 4, wobei der zweite Analysator bei jeder der mehreren Messungen die Urinprobe aus einem Probenbehälter ansaugt und die angesaugte Urinprobe misst.

7. Probenanalysesystem nach Anspruch 6, wobei der zweite Analysator als Reaktion auf eine Erfüllung einer vorbestimmten Abbruchbedingung, bevor der zweite Analysator eine letzte Aspiration von mehreren Aspirationen durchführt, eine Aspiration und eine Messung abbricht, die nach Erfüllung der vorbestimmten Abbruchbedingung durchgeführt werden sollen.

8. Probenanalysesystem nach Anspruch 7, wobei die vorbestimmte Abbruchbedingung darin besteht, dass mindestens eines der durch jede Messung durch den zweiten Analysator erhaltenen Messergebnisse eine vorbestimmte Nachweisbedingung erfüllt.

9. Probenanalysesystem nach Anspruch 7, wobei die vorbestimmte Abbruchbedingung darin besteht, dass ein vorbestimmter Analyt nachgewiesen wird.

10. Probenanalysesystem nach Anspruch 7, wobei die Verwaltungsvorrichtung eine Einstellung der vorbestimmten Abbruchbedingung empfängt.

11. Probenanalysesystem nach Anspruch 6, wobei der zweite Analysator die Urinprobe im Probenbehälter vor jeder Aspiration mehrerer Messungen bewegt.

12. Probenanalysesystem nach Anspruch 6, wobei der zweite Analysator jede der mehreren Messungen gemäß der gleichen Sequenz durchführt.

13. Probenanalysesystem nach Anspruch 6, wobei der zweite Analysator bei den mehreren Messungen die gleiche Menge der Urinprobe ansaugt.

14. Probenanalysesystem nach Anspruch 1, weiter umfassend eine Transportvorrichtung, die dazu konfiguriert ist, einen Probenbehälter, der die Urinprobe enthält, zum ersten und zum zweiten Analysator zu transportieren.

15. Probenanalysesystem nach Anspruch 1, wobei sowohl das erste Messergebnis als auch das zweite Messergebnis Ergebnisse bezüglich desselben Analyten voneinander sind.

16. Probenanalysesystem nach Anspruch 15, wobei der Analyt ein Leukozyt ist.

17. Probenanalysesystem nach Anspruch 1, wobei das erste Messergebnis ein Ergebnis bezüglich einer Menge eines ersten Analyten als der Analyt ist, und das zweite Messergebnis ein Ergebnis bezüglich einer Menge eines zweiten Analyten ist, der sich vom ersten Analyten unterscheidet.

18. Probenanalysesystem nach Anspruch 17, wobei der erste Analyt ein Protein ist, und der zweite Analyt mindestens eines von einem Guss, einem deformierten roten Blutkörperchen und einer Epithelzelle eines Nierentubulus ist.

19. Probenanalysesystem nach Anspruch 17, wobei der erste Analyt Hämoglobin ist, und der zweite Analyt ein rotes Blutkörperchen ist.

20. Probenanalysesystem nach Anspruch 1, wobei der zweite Analysator ein Urinpartikelanalysator ist, und der zweite Analysator die Urinprobe in einem Zeitraum misst, die der von der Verwaltungsvorrichtung bestimmten Probenmenge entspricht.

21. Probenanalyseverfahren unter Verwendung einer Vielzahl von Analysatoren, einschließlich eines ersten Analysators (10, 20) und eines zweiten Analysators (20, 30),
wobei der erste Analysator und der zweite Analysator unterschiedliche Messverfahren verwenden, umfassend:
Messen einer Urinprobe durch den ersten Analysator unter Verwendung eines Teststreifens, der seine Farbe entsprechend einer Menge eines Analyten in der Urinprobe ändert;
Erhalten eines ersten Messergebnisses durch den ersten Analysator, das die Menge des Analyten in drei oder mehr Niveaus basierend auf der Farbe des Teststreifens angibt,
wobei die Niveaus aus (i) einem ersten Niveau bestehen, der anzeigt, dass die Menge des Analyten ein normales Niveau ist, und (ii) einer Vielzahl von zweiten Niveaus bestehen, die anzeigen, dass die Menge des Analyten höher als das erste Niveau ist;
Erhalten des ersten Messergebnisses durch eine Verwaltungsvorrichtung (40);
Bestimmen durch die Verwaltungsvorrichtung einer Probenmenge, die durch den zweiten Analysator basierend auf dem durch das erste Messergebnis angezeigten Niveau der Menge des Analyten gemessen werden soll,
wobei die Probenmenge, die dem niedrigsten Niveau der Vielzahl von zweiten Niveaus entspricht, so konfiguriert werden kann, dass sie größer als die Probenmenge ist, die dem höchsten Niveau der Vielzahl von zweiten Niveaus entspricht; und
durch den zweiten Analysator, Messen der Urinprobe mit der durch die Verwaltungsvorrichtung bestimmten Probenmenge und Erhalten eines zweiten Messergebnisses der Urinprobe.

## Revendications

1. Système d'analyse d'échantillons (1) comprenant :
une pluralité d'analyseurs incluant un premier analyseur (10, 20) et un second analyseur (20, 30),
dans lequel le premier analyseur et le second analyseur utilisent des méthodes de mesure différentes, le premier analyseur étant configuré pour mesurer un échantillon d'urine en utilisant une bandelette réactive qui change de couleur selon une quantité d'un analyte dans l'échantillon d'urine et pour obtenir, sur la base de la couleur de la bandelette réactive, un premier résultat de mesure représentant la quantité de l'analyte en trois niveaux ou plus, et dans lequel les trois niveaux ou plus consistent en (i) un premier niveau indiquant que la quantité de l'analyte est un niveau normal, et (ii) une pluralité de seconds niveaux indiquant que la quantité de l'analyte est plus élevée que le premier niveau ; et
un dispositif de gestion (40) configuré pour obtenir le premier résultat de mesure et déterminer une quantité d'échantillon à mesurer par le second analyseur sur la base du niveau représenté par le premier résultat de mesure,
dans lequel la quantité d'échantillon correspondant au niveau le plus bas de la pluralité de seconds niveaux est configurable pour être supérieure à la quantité d'échantillon correspondant au niveau le plus élevé de la pluralité de seconds niveaux, et
dans lequel le second analyseur est configuré pour mesurer l'échantillon d'urine présentant la quantité d'échantillon déterminée par le dispositif de gestion et obtenir un second résultat de mesure de l'échantillon d'urine.

2. Système d'analyse d'échantillons selon la revendication 1, dans lequel le premier analyseur est un analyseur qualitatif d'urine et le second analyseur est un analyseur de particules d'urine.

3. Système d'analyse d'échantillons selon la revendication 1, dans lequel le dispositif de gestion obtient le second résultat de mesure et produit en sortie le premier résultat de mesure et le second résultat de mesure en association avec des informations d'identification de l'échantillon d'urine.

4. Système d'analyse d'échantillons selon la revendication 1, dans lequel le dispositif de gestion détermine, en tant que quantité d'échantillon à mesurer, un nombre de mesures effectuées sur l'échantillon d'urine par le second analyseur.

5. Système d'analyse d'échantillons selon la revendication 4, dans lequel le second analyseur obtient, lors de plusieurs mesures, le second résultat de mesure sur la base d'une pluralité de résultats de mesure de l'échantillon d'urine obtenus lors de plusieurs mesures.

6. Système d'analyse d'échantillons selon la revendication 4, dans lequel le second analyseur aspire, lors de chacune des plusieurs multiples, l'échantillon d'urine depuis un récipient à échantillon et mesure l'échantillon d'urine aspiré.

7. Système d'analyse d'échantillons selon la revendication 6, dans lequel le second analyseur annule, en réponse à une condition d'arrêt prédéterminée satisfaite avant que le second analyseur n'effectue une dernière aspiration de plusieurs aspirations, une aspiration et une mesure à effectuer après que la condition d'arrêt prédéterminée a été satisfaite.

8. Système d'analyse d'échantillons selon la revendication 7, dans lequel la condition d'arrêt prédéterminée est qu'au moins un des résultats de mesure obtenus par chaque mesure effectuée par le second analyseur satisfasse à une condition de détection prédéterminée.

9. Système d'analyse d'échantillons selon la revendication 7, dans lequel la condition d'arrêt prédéterminée est la détection d'un analyte prédéterminé.

10. Système d'analyse d'échantillons selon la revendication 7, dans lequel le dispositif de gestion reçoit un réglage de la condition d'arrêt prédéterminée.

11. Système d'analyse d'échantillons selon la revendication 6, dans lequel le second analyseur agite l'échantillon d'urine dans le récipient à échantillon avant chaque aspiration de plusieurs mesures.

12. Système d'analyse d'échantillons selon la revendication 6, dans lequel le second analyseur effectue chacune des plusieurs mesures selon une même séquence.

13. Système d'analyse d'échantillons selon la revendication 6, dans lequel le second analyseur aspire la même quantité de l'échantillon d'urine parmi les plusieurs mesures.

14. Système d'analyse d'échantillons selon la revendication 1, comprenant en outre un dispositif de transport configuré pour transporter un récipient à échantillon contenant l'échantillon d'urine vers le premier analyseur et le second analyseur.

15. Système d'analyse d'échantillons selon la revendication 1, dans lequel à la fois le premier résultat de mesure et le second résultat de mesure sont des résultats concernant le même analyte l'un par rapport à l'autre.

16. Système d'analyse d'échantillons selon la revendication 15, dans lequel l'analyte est un leucocyte.

17. Système d'analyse d'échantillons selon la revendication 1, dans lequel le premier résultat de mesure est un résultat concernant une quantité d'un premier analyte en tant qu'analyte, et le second résultat de mesure est un résultat concernant une quantité d'un second analyte différent du premier analyte.

18. Système d'analyse d'échantillons selon la revendication 17, dans lequel le premier analyte est une protéine et le second analyte est au moins l'un ou l'une d'un plâtre, d'un globule rouge déformé et d'une cellule épithéliale tubulaire rénale.

19. Système d'analyse d'échantillons selon la revendication 17, dans lequel le premier analyte est de l'hémoglobine et le second analyte est un globule rouge.

20. Système d'analyse d'échantillons selon la revendication 1, dans lequel le second analyseur est un analyseur de particules d'urine, et le second analyseur mesure l'échantillon d'urine dans un laps de temps correspondant à la quantité d'échantillon déterminée par le dispositif de gestion.

21. Méthode d'analyse d'échantillons utilisant une pluralité d'analyseurs incluant un premier analyseur (10, 20) et un second analyseur (20, 30),
dans lequel le premier analyseur et le second analyseur utilisent des méthodes de mesure différentes, comprenant :
la mesure, par le premier analyseur, d'un échantillon d'urine en utilisant une bandelette réactive qui change de couleur selon une quantité d'un analyte dans l'échantillon d'urine ;
l'obtention, par le premier analyseur, d'un premier résultat de mesure indiquant la quantité de l'analyte en trois niveaux ou plus, sur la base de la couleur de la bandelette réactive,
dans lequel les niveaux consistent en (i) un premier niveau indiquant que la quantité de l'analyte est un niveau normal, et (ii) une pluralité de seconds niveaux indiquant que la quantité de l'analyte est plus élevée que le premier niveau ;
l'obtention du premier résultat de mesure par un dispositif de gestion (40) ;
la détermination, par le dispositif de gestion, d'une quantité d'échantillon à mesurer par le second analyseur sur la base du niveau de la quantité de l'analyte indiquée par le premier résultat de mesure,
dans lequel la quantité d'échantillon correspondant au niveau le plus bas de la pluralité de seconds niveaux est configurable pour être supérieure à la quantité d'échantillon correspondant au niveau le plus élevé de la pluralité de seconds niveaux ; et
par le second analyseur, la mesure de l'échantillon d'urine présentant la quantité d'échantillon déterminée par le dispositif de gestion et l'obtention d'un second résultat de mesure de l'échantillon d'urine.
